(19) Europäisches Patentamt
European Patent Office
Office européen des brevets

(11) EP 3 488 772 B1

(12) # EUROPEAN PATENT SPECIFICATION

(45) Date of publication and mention of the grant of the patent:
**12.07.2023 Bulletin 2023/28**

(21) Application number: **18208987.0**

(22) Date of filing: **28.11.2018**

(51) International Patent Classification (IPC):
***A61B 5/00*** *(2006.01)* ***A61B 5/339*** *(2021.01)*

(52) Cooperative Patent Classification (CPC):
**A61B 5/7203; A61B 5/0006; A61B 5/339;**
A61B 5/7267; A61B 2505/07

(54) **ECG DATA CLASSIFICATION FOR USE IN FACILITATING DIAGNOSIS OF CARDIAC RHYTHM DISORDERS WITH THE AID OF A DIGITAL COMPUTER**

EKG-DATENKLASSIFIZIERUNG ZUR VERWENDUNG BEI DER ERLEICHTERUNG DER DIAGNOSE VON HERZRHYTHMUSSTÖRUNGEN MIT HILFE EINES DIGITALEN COMPUTERS

CLASSIFICATION DE DONNÉES D'ECG POUR FACILITER LE DIAGNOSTIC DE TROUBLES DU RYTHME CARDIAQUE À L'AIDE D'UN ORDINATEUR NUMÉRIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.11.2017 US 201762591715 P**
**23.03.2018 US 201815934888**

(43) Date of publication of application:
**29.05.2019 Bulletin 2019/22**

(73) Proprietor: **Bardy Diagnostics, Inc.**
**Seattle, WA 98104 (US)**

(72) Inventors:
- **BARDY, Gust H.**
  **Carnation, WA Washington 98014 (US)**
- **DREISBACH, Ezra M.**
  **Vashon, WA Washington 98070 (US)**

(74) Representative: **Murgitroyd & Company**
**Murgitroyd House**
**165-169 Scotland Street**
**Glasgow G5 8PL (GB)**

(56) References cited:
**US-A1- 2017 196 472**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 488 772 B1

## Description

### TECHNICAL FIELD

This application relates in general to electrocardiographic monitoring and, in particular, to a system and method for ECG data classification for use in facilitating diagnosis of cardiac rhythm disorders with the aid of a digital computer.

### BACKGROUND ART

An ECG procedure measures cardiac electrical potentials that can be graphed to visually depict the electrical activity of the heart over time and allows physicians to diagnose cardiac function by visually tracing the cutaneous electrical signals (action potentials) that are generated by the propagation of the transmembrane ionic currents that trigger the depolarization of cardiac fibers. Conventionally, a standardized set format 12-lead configuration is used by an ECG machine to record cardiac electrical signals from well-established traditional chest locations.

An ECG trace contains alphabetically-labeled waveform deflections, PQRSTU, that represent distinct features within the cyclic cardiac activation sequence and that can be interpreted post-ECG recordation to derive heart rate and physiology and for use in medical diagnosis and treatment. The P-wave represents atrial depolarization, which causes atrial contraction. The QRS-complex represents ventricular depolarization and is the largest electrical signal of the heart. The T-wave and U-wave represents ventricular repolarization. The T and U-waves are not usually used in diagnosing most cardiac rhythm disorders and are included for completeness.

Here, the focus is around the R-wave, which is often used as an abbreviation for the QRS-complex. When measuring the time between an R-R interval, one can get a beat-by-beat assessment of heart rate. Typically, the R-R interval span between successive R-waves, in a normal heart, is 600 milliseconds (ms) to 1000 ms (i.e., one second) long, which respectively corresponds to 100 to 60 beats per minute (bpm). If one further considers the R-R interval as occurring over time, beat-by-beat, detailed cardiac physiology data may be embedded to provide information that allows a physician to understand, at a glance, the context of the associated ECG rhythm both before and after a suspected rhythm abnormality and can be of confirmational and collaborative value in cardiac arrhythmia diagnosis and treatment. (mm) per second. A lower speed is not recommended because ECG graph resolution degrades at lower speeds and diagnostically-relevant features may be lost. Conversely, a half-hour ECG recording, progressing at 25 mm/s, results in 45 meters of ECG waveforms that, in printed form, is cumbersome and, in electronic display form, will require significant back and forth toggling between pages of waveforms, as well as presenting voluminous data transfer and data storage concerns. As a result, ECGs are less than ideal tools for diagnosing cardiac arrhythmia patterns that only become apparent over an extended time frame, such as 10 minutes or longer.

In addition to or in lieu of physician review, the R-wave data can be provided to analysis software for identification of cardiac events and patient diagnosis. However, such software generally operates best on datasets that represent a few minutes to two days of ECG data. As newer ECG monitoring devices enter the market, many include longer data recording periods. Accordingly, such analysis programs are overloaded with data, which can hinder effectiveness of the analysis, leading to an incorrect patient diagnosis.

Further, performing an analysis of data that includes large amounts of noise can affect accuracy of patient diagnosis. For instance, noise can mask portions of the ECG data, which can prevent analysis software or a medical professional from identifying patterns of cardiac events, as well as lead to an incorrect diagnosis or lack of a diagnosis.

Therefore, a need remains for improving quality of ECG data, while reducing amounts of the ECG data for analysis, including patient diagnosis, by identifying and removing noise. Such noise can be recognized by differentiating regions of valid ECG data from noise using an adaptive noise detector and the regions of noise can be removed from the valid ECG data.

US2017196472A1 discloses a system and method for secure physiological data processing and delivery are provided. A log of identifiers of physiological monitoring patches and of passwords is maintained in a secure database. Physiological monitoring data obtained using one of the patches is received by at least one server together with the identifier and the password associated with that patch. The received identifier and password are compared with the identifiers and passwords stored in the log. The received physiological monitoring data is stored in electronic medical records associated with the matching identifier.

### SUMMARY

The present application provides a system and method for ECG data classification for use in facilitating diagnosis of cardiac rhythm disorders with the aid of a digital computer, in accordance with the claims which follow.

DESCRIPTION OF THE DRAWINGS

FIGURE 1 is a graph showing, by way of example, a single ECG waveform.

FIGURE 2 is a graph showing, by way of example, a prior art Poincaré R-R interval plot.

FIGURE 3 is a flow diagram showing a method for facilitating diagnosis of cardiac rhythm disorders with the aid of a digital computer in accordance with one embodiment.

FIGURE 4 is a flow diagram showing a routine for constructing and displaying a diagnostic composite plot for use in the method of FIGURE 3.

FIGURE 5 is a flow diagram showing a routine for constructing an extended-duration R-R interval plot for use in the routine of FIGURE 4.

FIGURE 6 is a diagram showing, by way of example, a diagnostic composite plot generated by the method of FIGURE 3.

FIGURE 7 is a diagram showing, by way of example, a diagnostic composite plot for facilitating the diagnosis of sinus rhythm (SR) transitioning into atrial fibrillation (AF).

FIGURE 8 is a diagram showing, by way of example, a diagnostic composite plot for facilitating the diagnosis of 3:1 atrial flutter (AFL) transitioning into SR.

FIGURE 9 is a diagram showing, by way of example, a diagnostic composite plot for facilitating the diagnosis of atrial trigeminy.

FIGURE 10 is a diagram showing, by way of example, a diagnostic composite plot for facilitating the diagnosis of maximum heart rate in an episode of AF during exercise.

FIGURE 11 is a diagram showing, by way of example, a diagnostic composite plot for facilitating the diagnosis of SR transitioning into AFL transitioning into AF.

FIGURE 12 is a diagram showing, by way of example, a diagnostic composite plot for facilitating the diagnosis of sinus tachycardia and palpitations that occurred during exercise accompanied by a jump in heart rate.

FIGURE 13 is a diagram showing, by way of example, a diagnostic composite plot for facilitating the diagnosis of bradycardia.

FIGURE 14 is a block diagram showing a system for facilitating diagnosis of cardiac rhythm disorders with the aid of a digital computer in accordance with one embodiment.

FIGURE 15 is a perspective view showing an ECG ambulatory monitor having an extended wear electrode patch with a monitor recorder inserted.

FIGURE 16 is a perspective view showing the monitor recorder of FIGURE 15. FIGURE 17 is a perspective view showing the extended wear electrode patch of FIGURE 15 without a monitor recorder inserted.

FIGURE 18 is a bottom plan view of the monitor recorder of FIGURE 15. FIGURE 19 is a flow diagram showing a method for ECG data classification for use in facilitating diagnosis of cardiac rhythm disorders with the aid of a digital computer.

FIGURE 20 is a flow diagram showing a method for noise detection.

FIGURE 21 is a flow diagram showing, by way of example, a method for training an adaptive noise detector.

FIGURE 22 is a flow diagram showing, by way of example, a method for classification of ECG data.

FIGURE 23 is a flow diagram showing, by way of example, a method for trimming data segments classified as noise.

FIGURE 24 is a diagram showing, by way of example, a diagnostic composite plot showing data from a button press window.

FIGURE 25 is a diagram showing, by way of example, a summary graph of data press occurrences.

BEST MODE FOR CARRYING OUT THE INVENTION

A normal healthy cardiac cycle repeats through an expected sequence of events that can be visually traced through an ECG. Each cycle starts with cardiac depolarization originating high in the right atrium in the sinoatrial (SA) node before spreading leftward towards the left atrium and inferiorly towards the atrioventricular (AV) node. After a delay in the AV node, the depolarization impulse transits the Bundle of His and moves into the right and left bundle branches and Purkinje fibers to activate the right and left ventricles.

When a rhythm disorder is suspected, diagnostically-relevant arrhythmic events in the cardiac cycle can often be identified and evaluated with the assistance of an ECG and R-R interval tachography, somewhat similar to Poincaré plots. Routine ECG evaluation is primarily focused on identifying changes to expected ECG waveform shapes. R-R interval tachographs are focused on showing heart rate trends prior to and after an arrhythmia. This information is critical to understanding the underpinnings of arrhythmia onset and offset, both features critical to managing the patient regardless of the specific arrhythmia shown on the ECG. FIGURE 1 is a graph showing, by way of example, a single ECG waveform 10. The x-axis represents approximate time in units of tenths of a second and the y-axis represents approximate cutaneous electrical signal strength in units of millivolts. By long-standing convention, ECGs are typically printed or displayed at an effective paper speed of 25 millimeters (mm) per second. Although in practice an ECG may be provided to a physician in traditional paper-printed form, in "virtual" electronic display form, or both. Nevertheless, the term "effective paper speed" is still widely applied as a metric to normalize the recorded ECG signal to a standardized grid of 1 mm squares (omitted for the sake of clarity in FIGURE 1), whereby each 1 mm horizontal box in the grid corresponds to 0.04 s (40 ms) of recorded time. Other effective paper speeds, grid sizes and units of display are possible.

A full ECG consists of a stream of alphabetically-labeled waveforms 10 that collectively cover cardiac performance over a period of observation. For a healthy patient, within each ECG waveform 10, the P-wave 11 will normally have a smooth, normally upward, positive waveform that indicates atrial depolarization. The QRS complex 17 will usually follow during normal rhythms, often with a downward deflection of a Q-wave 12, followed by a larger upward deflection of an R-wave 13, and be terminated with a downward waveform of the S-wave 14, which are collectively representative of ventricular depolarization. This term, QRS, is often reduced to the "R-wave". Thus, reference to the R-R interval means the time from one R-wave (or QRS) to another R-wave. For completeness sake of showing the full ECG, we identify the T-wave 15, which will normally be a modest upward waveform, representative of ventricular repolarization, while the U-wave 16, which is often not directly observable, will indicate the recovery period of the Purkinje conduction fibers. For the purposes of the R-R plot, this part of the ECG waveform will not be pertinent.

Rhythm disorders often manifest through R-R interval variability or through the patterns formed by R-R intervals over an extended time period prior to and after a specific arrhythmia onset and offset. Both are important tools in the diagnosis of cardiac rhythm abnormalities. For example, atrial fibrillation (AF) is the chaotic firing of the atria that leads to an erratic activation of the ventricles. AF is initially diagnosed by an absence of organized P-waves 11 and confirmed by erratic ventricular rates that manifest in an ECG R-R interval plot as a cloud-like pattern of irregular R-R intervals due to an abnormal conduction of impulses to the ventricles. There is a Gaussian-like distribution to these R-R intervals during AF. Similarly, atrial flutter (AFL) is an abnormal heart rhythm in which cardiac impulses travel along pathways within the right atrium in an organized circular motion, causing the atria to beat faster than and out of sync with the ventricles. During AFL, the heart beats quickly, yet with a regular pattern. Although AFL presents in an ECG in a "sawtooth" pattern, AFL behavior can fluctuate wildly. Such fluctuation can be confirmed in an ECG by characteristic R-R interval patterns that usually manifest as 2:1 atrioventricular (AV) conduction, 4:1 AV conduction or even variable AV conduction, as it frequently can be. The importance of this latter manifestation of AFL relates to how AFL can be distinguished from AF. The R-R interval plot can clearly distinguish the two unlike algorithms that frequently conflate AF with AFL and variable conduction.

Conventionally, R-R intervals have been visualized using Poincaré plots. FIGURE 2 is a graph showing, by way of example, a prior art Poincaré R-R interval plot 18. The x-axis represents the duration of R-R interval n in units of milliseconds (ms). The y-axis represents the duration of R-R interval n + 1 also in units of ms. Ordinarily, the x- and y-axes use the same units, so as to form a trend line 19 along the 45-degree angle. When an R-R interval is equal to the successive R-R interval, as often occurs when heart rhythm is regular, the dot representing the two intervals falls onto the 45-degree trend line 19. Conversely, when an R-R interval has changed since the preceding R-R interval, the dot representing the two intervals falls off the 45-degree trend line 19 and, as the difference between successive R-R intervals increases, the dots fall further away from the trend line 19.

The number of dots deviating from the trend line 19 in a Poincaré plot can indicate the frequency of occurrence of irregular heartbeats when compared to the number of dots on the trend line 19. The distance of the dots to the trend line 19 can approximate the extent of heart rate change from one heartbeat to the next. However, as heart rate change is limited to only successively-occurring heartbeats, the linearity of time and associated contextual information over an extended time frame are lost. Poincare plots can be generally predictive but they have limited value when used to understand physiological antecedents to an arrhythmia pre- and post-rhythm events, for example something as simple as exercise. In addition, significant changes in heart rate, particularly spikes in heart rate, such as due to sinus rhythm transitions to atrial ) flutter, may be masked, distorted or even omitted in a Poincaré plot if the change occurs over non-successive heartbeats. In summary, a Poincaré plot is more useful as a mathematical tool than a physiological one, and therefore a Poincaré plot cannot truly represent what the heart is doing serially over time with respect to changes in the heart's normal and abnormal physiology on a beat-by-beat basis.

Despite the limitations of Poincaré plots and related forms of R-R interval tachography, R-R interval data when presented in a format duplicating temporal physiological events manifest on an actual ECG remains a key tool that physicians can rely upon to identify temporally-related cardiac dysrhythmic patterns. Interpretation of R-R interval data can be assisted by including multiple temporal points of reference and a plot of R-R interval data that comparatively depicts ) heart rate variability in concert with R-R interval data. FIGURE 3 is a flow diagram showing a method 20 for facilitating diagnosis of cardiac rhythm disorders with the aid of a digital computer in accordance with one embodiment. The method 20 can be implemented in software and execution of the software can be performed on a computer, such as further described infra with reference to FIGURE 14, as a series of processes or method modules or steps.

As a precursor step, the patient's ECG data are recorded over a set time period (step 21), which can be over a short term or extended time frame. ECG recordation, as well as other physiological monitoring, can be provided through various kinds of ECG-capable monitoring ensembles, including a standardized 12-lead ECG setup, such as used for clinical ECG monitoring, a portable Holter-type ECG recorder for traditional ambulatory ECG monitoring, or ) a wearable ambulatory ECG monitor, such as a flexible extended wear electrode patch and a removable reusable (or single use) monitor recorder, such as described in commonly-assigned U.S. Patent No. 9,345,414, issued May 24, 2016. The wearable ambulatory ECG monitor includes an electrode patch and monitor recorder that are synergistically optimized to capture electrical signals from the propagation of low amplitude, relatively low frequency content cardiac action potentials, particularly the P-waves, generated during atrial activation, and is described in further detail below with respect to FIGURES 15. Still other forms of ECG monitoring assemblies are possible.

Upon completion of the monitoring period, the ECG and any other physiological data are downloaded or retrieved into a digital computer, as further described infra with reference to FIGURE 14, with, for instance, the assistance of a download station or similar device, or via wireless connection, if so equipped, and a vector of the downloaded or retrieved ECG data is obtained (step 22). In one embodiment, the vector of ECG data represents a 40-minute (or other duration) time span that is used in constructing the plot of R-R interval data, although other pre-event and post-event time spans are possible. Optionally, a potentially-actionable cardiac event within the vector of ECG data can be identified and the ECG data during, prior to and after the event is selected (step 23). The event could be identified with the assistance of a software package, such as Holter LX Analysis Software, licensed by NorthEast Monitoring, Inc., Maynard, MA; IntelliSpace Cardiovascular Image and Information management system, licensed Koninklijke Philips N.V., Amsterdam, Netherlands; MoMe System, licensed by InfoBionic, Lowell, MA; Pyramis ECG Management, licensed by Mortara Instrument Inc., Milwaukee, WI; ICS Clinical Suite, licensed by Spacelabs Healthcare Inc., Snoqualmie, WA; or a customized software package. Alternatively, the potentially-actionable cardiac event could be identified by a physician or technician during review of the ECG data.

To improve diagnosis of heart rate variability, a diagnostic composite plot is constructed that includes one or more temporal points of reference into the ECG data, which provide important diagnostic context, and a plot of R-R interval data is constructed based on the vector of ECG data (step 24), as further described infra with reference to FIGURE 4. Briefly, both near field and far field contextual views of the ECG data are constructed and displayed. Both views are temporally keyed to an extended duration R-R interval data view that, in one embodiment, is scaled non-linearly (for practical viewing reasons) to maximize the visual differentiation for frequently-occurring heart rate ranges, such that a single glance allows the physician to make a diagnosis. The use of a non-linear heart rate scale adjacent to the ECG prevents a skewing of the R-R data to exceed the visual format of a page of paper. Thus, all three views are presented simultaneously, in a visually convenient format that can present 3 sequential ECG data sets per page, thereby allowing the interpreting physician to diagnose rhythm and the pre- and post-contextual events leading up to a cardiac rhythm of interest in a reading and storage friendly manner.

In a further embodiment, findings made through interpretation of heart rate variability patterns in the diagnostic composite plot can be analyzed to form a diagnosis of a cardiac rhythm disorder (step 25), such as the cardiac rhythm disorders listed, by way of example, in Table 1. For instance, the heart rate variability patterns in the diagnostic composite plot could be provided to a system that programmatically detects AF by virtue of looking for the classic Gaussian-type distribution on the "cloud" of heart rate variability formed in the plot of R-R interval data, which can be corroborated by the accompanying contextual ECG data. Finally, therapy to address diagnosed disorder findings can optionally be programmed into a cardiac rhythm therapy delivery device (step 26), such as an implantable medical device (IMD) (not shown), including a pacemaker, implantable cardioverter defibrillator (ICD), or similar devices.

| **Cardiac Rhythm Disorders** |
|---|
| ▪ Normal sinus rhythm |
| ▪ Sinus Bradycardia |
| ▪ Sinus Tachycardia |
| ▪ Premature atrial and ventricular beats |
| ▪ Ectopic atrial tachycardia |
| ▪ Atrial fibrillation |
| ▪ Atrial flutter |
| ▪ Atrial or ventricular bigeminy, trigeminy or quadrigeminy |
| ▪ Sinus Bradycardia |

(continued)

| Cardiac Rhythm Disorders |
|---|
| ▪ Fusion beats |
| ▪ Interpolated ventricular premature beats |
| ▪ Intraventricular conduction delay |
| ▪ Junctional rhythm |
| ▪ AV Nodal re-entrant tachycardia |
| ▪ AV re-entrant tachycardia |
| ▪ Wolff-Parkinson-White Syndrome and Pre-excitation |
| ▪ Ventricular tachycardia |
| ▪ Accelerated idioventricular rhythm |
| ▪ AV Wenckebach block |
| ▪ AV Type II block |
| ▪ Sinoatrial block |

**Table 1.**

A diagnostic composite plot is constructed and displayed to help physicians identify and diagnose temporally-related cardiac dysrhythmic patterns. The diagnostic composite plot includes ECG traces from two or more temporal points of reference and a plot of R-R interval data, although other configurations of ECG data plots when combined with the R-R interval plot will also provide critical information. FIGURE 4 is a flow diagram showing a routine 30 for constructing and displaying a diagnostic composite plot for use in the method 20 of FIGURE 3. Specific examples of diagnostic composite plots are discussed in detail infra with reference to FIGURES 7-13.

In the diagnostic composite plot, R-R interval data is presented to physicians in a format that includes views of relevant near field and far field ECG data, which together provide contextual information that improves diagnostic accuracy. In a further embodiment, other views of ECG data can be provided in addition to or in lieu of the near field and far field ECG data views. The near field (or short duration) ECG data provides a "pinpoint" classical view of an ECG at traditional recording speed in a manner that is known to and widely embraced by physicians. This near field ECG data is coupled to a far field (or medium duration) ECG data view that provides an "intermediate" lower resolution, offering pre- and post-event contextual ECG data. Thus, the extended-duration R-R interval plot is first constructed (step 31), as further described infra with reference to FIGURE 5. Optionally, noise can be filtered from the R-R interval plot (step 32), which is then displayed (step 33). In a further embodiment, noise can be filtered from the ECG data prior to constructing the R-R interval plot, as further described below with reference to FIGURE 19. Noise filtering can include low-pass or high-pass filtering or other forms of signal processing, including automatic gain control, such as described in commonly-assigned U.S. Patent No. 9,345,414, issued May 24, 2016, cited supra, as well as identification via an adaptive noise detector, as further described in detail below with respect to FIGURES 19-25.

Rhythm disorders have different weightings depending upon the context with which they occur. In the diagnostic composite plot, the R-R interval data view and the multiple views of the ECG data around which the R-R data embrace, provide that necessary context. Effectively, the short and medium duration ECG data that accompanies the extended-duration R-R interval plot represents the ECG data "zoomed" in around a temporal point of reference identified in the center (or other location) of the R-R interval plot, thereby providing a short-term, medium-term and long-term visual context to the physician that allows temporal assessment of cardiac rhythm changes in various complementary views of the heart’s behavior. The durations of the classical "pinpoint" view, the pre- and post-event "intermediate" view, and the R-R interval plot are flexible and adjustable. In one embodiment, the diagnostic composite plot displays R-R interval data over a forty-minute duration and ECG data over short and medium durations (steps 34 and 35), such as four-second and 24-second durations that provide two- and 12-second segments of the ECG data before and after the RR interval plot’s temporal point of reference, which is generally in the center of the R-R interval plot, although other locations in the R-R interval plot could be identified as the temporal point of reference. The pinpoint "snapshot" and intermediate views of ECG data with the extended term R-R interval data comparatively depicts heart rate context and patterns of behavior prior to and after a clinically meaningful arrhythmia or patient concern, thereby enhancing diagnostic specificity of cardiac rhythm disorders and providing physiological context to improve diagnostic ability. In a further embodiment, diagnostically relevant cardiac events can be identified and the R-R interval plot can be constructed with a cardiac event centered in the middle (or other location) of the plot, which thereby allows pre- and post-event heart rhythm data to be contextually "framed" through the pinpoint and intermediate ECG data views. Other durations, intervals and presentations of ECG data are possible, including allowing the physician or viewer to select or scroll through the raw ECG data to advance the ECG data together with the R-R interval data, allowing context to be selected by the viewer.

The extended-duration R-R interval plot presents beat-to-beat heart rate variability in a format that is intuitive and contextual, yet condensed. The format of the R-R interval plot is selected to optimize visualization of cardiac events in a compressed, yet understandable field of view, that allows for compact presentation of the data akin to a cardiologists understanding of clinical events. FIGURE 5 is a flow diagram showing a routine 40 for constructing an extended-duration R-R interval plot for use in the routine 30 of FIGURE 4. The duration of the R-R interval plot can vary from less than one minute to the entire duration of the recording. Thus, a plurality of R-wave peaks is first selected out of the vector of ECG data (step 41) appropriate to the duration of the R-R interval plot to be constructed. For successive pairs of the R-wave peaks (steps 42-43), the difference between the recording times of the R-peaks is calculated (step 43). Each recording time difference represents the length of one heartbeat. The heart rate associated with the recording time difference is determined by taking an inverse of the recording time difference and normalizing the inverse to beats per minute (step 44). Taking the inverse of the recording time difference yields a heart rate expressed in beats per second, which can be adjusted by a factor of 60 to provide a heart rate expressed in bpm. Calculation of the differences between the recording times and the associated heart rate continues for all of the remaining pairs of the R-wave peaks (step 44).

The pairings of R-R intervals and associated heart rates are formed into a two-dimensional plot. R-R intervals are plotted along the x-axis and associated heart rates are plotted along the y-axis. The range and scale of the y-axis (heart rate) can be adjusted according to the range and frequency of normal or patient-specific heart rates, so as to increase the visual distinctions between the heart rates that correspond to different R-R intervals. In one embodiment, the y-axis of the R-R interval plot has a range of 20 to 300 beats per minute and R-R intervals corresponding to heart rates falling extremely outside of this range are excluded to allow easy visualization of 99+% of the heart rate possibilities. To encompass all heart rates theoretically possible, 0 to 350 bpm, would alter the practical visibility of the interval range for the vast majority of events. (Note that these extreme rates remain visible in the ECG.).

In a further embodiment, the y-axis has a non-linear scale that is calculated as a function of the x-axis (R-R interval), such that:

$$y = \left(\frac{x - \min bpm}{\max bpm - \min bpm}\right)^n$$

where x is the time difference, min *bpm* is the minimum heart rate, max *bpm* is the maximum heart rate, and $n < 1$. The non-linear scale of the y-axis accentuates the spatial distance between successive heart rates when heart rate is low. For example, when $n = 2$, the spatial difference between 50 and 60 bpm is 32% larger than the spatial difference between 90 bpm and 100 bpm, and 68% larger than the spatial difference between 150 bpm and 160 bpm. As a result the overall effect is to accentuate the spatial differences in frequently-occurring ranges of heart rate and deemphasize the spatial differential in ranges of heart rate where a deviation from norm would have been apparent, thus maximizing the spatial efficiency in data presentation. The goal is to show cardiac events in a simple, small visual contextual format. Larger scales and larger formats bely the practical limits of single-page presentations for the easy visualization at a glance by the busy physician. The visual distinctions between the heart rates that correspond to different R-R intervals stand out, especially when plotted on a non-linear scale. In this configuration, three distinct arrhythmia events can be presented in standard 8.5 by 11 inch page formats. However, other formats are possible. Also, other y-axis ranges and scales are possible as may be selected by distinct clinical needs and specific diagnostic requirements.

The diagnostic composite plot includes a single, long range view of R-R interval data and a pair of pinpoint ECG data views that together help to facilitate rhythm disorder diagnosis by placing focused long-term heart rate information alongside short-term and medium-term ECG information. Such pairing of ECG and R-R interval data is unique in its ability to inform the physician of events prior to, during and after a cardiovascular event. FIGURE 6 is a diagram showing, by way of example, a diagnostic composite plot 50 generated by the method 30 of FIGURE 3. Note that the diagnostic composite plot can be tailored to include more than one view of R-R interval data and as many views of contextual ECG data as needed. In a further embodiment, a background information plot presenting an extended far field of related information can be included, such as activity amount, activity intensity, posture, syncope impulse detection, respiratory rate, blood pressure, oxygen saturation (SpO2), blood carbon dioxide level (pCO2), glucose, lung wetness, and temperature. Other forms of background information are possible. In a still further embodiment, background information can be layered on top of or keyed to the diagnostic composite plot 50, particularly at key points of time in the R-R interval data plot, so that the context provided by each item of background information can be readily accessed by the reviewing physician.

The diagnostic composite plot 50 includes an ECG plot presenting a near field (short duration) view 51, an ECG plot presenting an intermediate field (medium duration) view 52, and an R-R interval data plot presenting a far field (extended duration) view 53. The three views 51, 52, 53 are juxtaposed alongside one other to allow quick back and forth referencing of the full context of the heart's normal and abnormal physiology. Typically, a temporal point of reference, which could be a diagnostically relevant cardiac event, patient concern or other indicia, would be identified and centered on the x-axis in all three views. The placement of the temporal point of reference in the middle of all three x-axes enables the ECG data to be temporally keyed to the R-R interval data appearing in the center 60 of the R-R interval data view 53, with a near field view 51 of an ECG displayed at normal (paper-based) recording speed and a far field view 52 that presents the ECG data occurring before and after the center 60. As a result, the near field view 51 provides the ECG data corresponding to the R-R interval data at the center 60 (or other location) in a format that is familiar to all physicians, while the intermediate field view 52 enables presentation of the broader ECG data context going beyond the borders of the near field view 51. In a further embodiment, the center 60 can be slidably adjusted backwards and forwards in time, with the near field view 51 and the far field view 52 of the ECG data automatically or manually adjusting accordingly to stay in context with the R-R interval data view 51. In a still further embodiment, multiple temporal points of reference can be identified with each temporal point of reference being optionally accompanied by one or more dedicated sets of ECG data views.

The collection of plots are conveniently arranged close enough to one another to facilitate printing on a single page of standard sized paper (or physical paper substitute, such as a PDF file), although other layouts of the plots are possible. The far field view 53 is plotted with time in the x-axis and heart rate in the y-axis. The R-R intervals are calculated by measuring the time occurring between successive R-wave peaks. In one embodiment, the far field view 53 presents R-R interval data (expressed as heart rate in bpm) that begins about 20 minutes prior to and ends about 20 minutes following the center 60, although other durations are possible.

The near field view 51 and intermediate field view 52 present ECG data relative to the center 60 of the far field view 53. The near field view 51 provides a pinpoint or short duration view of the ECG data. In one embodiment, the near field view 51 presents ECG data 55 that begins about two seconds prior to and ends about two seconds following the center 60, although other durations are possible. The intermediate field view 52 provides additional contextual ECG information allowing the physician to assess the ECG itself and gather a broader view of the rhythm before and after a "blow-up" of the specific arrhythmia of interest. In one embodiment, the intermediate field view 52 presents ECG data 56 that begins about 12 seconds prior to and ends about 12 seconds following the center 60, although other durations are possible. For convenience, the eight-second interval of the ECG data 56 in the intermediate field view 52 that makes up the ECG data 56 in the near field view 51 is visually highlighted, here, with a surrounding box 57. In addition, other views of the ECG data, either in addition to or in lieu of the near field view 51 and the far field view 52 are possible. Optionally, an ECG plot presenting an extended far field view 54 of the background information can be included in the diagnostic composite plot 50. In one embodiment, the background information is presented as average heart rate with day and night periods 58 alternately shaded along the x-axis. Other types of background information, such as activity amount, activity intensity, posture, syncope impulse detection, respiratory rate, blood pressure, oxygen saturation (SpO2), blood carbon dioxide level (pCO2), glucose, lung wetness, and temperature, are possible.

Examples of the diagnostic composite plot as applied to specific forms of cardiac rhythm disorders will now be discussed. These examples help to illustrate the distinctive weightings that accompany different forms of rhythm disorders and the R-R interval and ECG waveform deflection context with which they occur. FIGURE 7 is a diagram showing, by way of example, a diagnostic composite plot 70 for facilitating the diagnosis of sinus rhythm (SR) transitioning into AF. SR is indicated through the presence of a reasonably steady baseline, but with subsidiary lines of premature beats and their compensatory pauses. SR manifests as a shadowing 71 of a high heart rate line and a low heart rate line. AF is characterized by irregular heartbeats with a somewhat random variation of R-R intervals, although within a limited range and concentrating in a Gaussian-like distribution pattern around a mean that varies over time. Such characteristics can also be distinct from the more random nature of noise. Although AF can be diagnosed by viewing a near field view 51 of ECG data showing heartbeats with reversed P-wave and irregular R-R intervals, this approach may be unclear when viewing "snippets" of ECG data, especially when associated with poor quality ECG signals. The presence of AF can also be confirmed through a far field view 53 of R-R interval data, in which the R-R intervals assume superficially appearing disorganized, spread-out and decentralized scattered cloud 72 along the x-axis, in comparison to a concentrated, darkened line typical of a more organized cardiac rhythm.

FIGURE 8 is a diagram showing, by way of example, a diagnostic composite plot 80 for facilitating the diagnosis of 3:1 atrial flutter (AFL) transitioning into SR with frequent premature ectopic atrial beats. In the initial part of the R-R interval plot, the R-R intervals have a discernible aggregated line in the middle of the cloud 81 when the rhythm has yet to stabilize into a set pattern, not quite AF and not quite AFL. Immediately thereafter, a dense line representing firm 3:1 atrial flutter stabilizes the rhythm prior to the transition into SR associated with the presence of two seesawing baselines that result from frequent atrial ectopy causing short coupling intervals and then compensatory long coupling intervals. SR is indicated by the middle of the three lines with a low heart rate line consistent with the compensatory pause (long coupling interval) and a high heart rate line with the shortest coupling interval representing the series of atrial premature beats 82, and thus, at a faster heart rate.

FIGURE 9 is a diagram showing, by way of example, a diagnostic composite plot 90 for facilitating the diagnosis of atrial trigeminy. Atrial trigeminy is characterized by three heartbeat rates appearing intermittently yet reasonably regularly. Although atrial trigeminy can be diagnosed by viewing a near field view 51 of ECG data, the pattern and its clinical impact and frequency is significantly more recognizable in a far field view 53 of R-R interval data, in which a repeating pattern of three distinct heartbeat lines are persistently present and clearly visible 91. This view also provides the physician with a qualitative feel for the frequency of the event troubling the patient that is not discernible from a single ECG strip.

FIGURE 10 is a diagram showing, by way of example, a diagnostic composite plot 100 for facilitating the diagnosis of maximum heart rate in an episode of AF during exercise. In a far field view 50 of R-R interval data, AF manifests through a dispersed cloud of dots (Gaussian-like distribution) without a discernible main heart rate line representing regular heartbeats 101. Under exercise, the maximum heartbeat can be located by an increase in heart rate clustered about the cloud 102. In addition, individual dots above the 200 bpm range throughout the entire 40-minute range indicates the maximum heart rate during exercise. The very rapid rise in heart rate can be critical to patient management, as such bumps in rate by exercise can prove dangerous and even trigger cardiac arrest by inducing ventricular fibrillation. Their very presence is easily visualized in the R-R interval data plot, thereby allowing the physician to alter therapy sufficiently to control such potentially damaging rises in heart rate.

FIGURE 11 is a diagram showing, by way of example, a diagnostic composite plot 110 for facilitating the diagnosis of SR transitioning into AFL transitioning into AF. In a far field view 53 of R-R interval data, SR manifests as an uneven main heart rate line with a fluctuating height 111. At the onset of AFL, the main heart rate line breaks away at a lower heart rate than the SR main heart rate line 112. The episode of AFL further evolves into AF as characterized by a dispersed cloud of irregular heartbeats without concentrated heart rate lines 113. This view provides critical information to the physician managing AF patients in that, at a glance, the view provides data that tells the physician that the patient's AF may be the consequence of AFL. Such knowledge may alter both drug and procedure therapies, like catheter ablation details of intervention. These two disorders are not interchangeable and generally demand different drug and different catheter ablation approaches.

FIGURE 12 is a diagram showing, by way of example, a diagnostic composite plot 120 for facilitating the diagnosis of sinus tachycardia and palpitations that occurred during exercise accompanied by a jump in heart rate. In a far field view 50 of R-R interval data, sinus tachycardia is indicated by the presence of a baseline heart rate of about 60 bpm 121 that spikes up to around 100 bpm 122 and gradually slopes down with a wide tail 123, reflecting a sharp rise of heart rates followed by a gradual decline. The associated ECG data in the near field and intermediate field views (not shown) can confirm the rhythm as sinus rhythm and a normal response to exercise. This rhythm, although superficially obvious, was associated with symptoms of palpitations and demonstrates a sensitivity to heart rate fluctuations, rather than a sensitivity to an arrhythmia. This common problem is often dismissed as merely sinus tachycardia, rather than recognizing the context of a changing rate that generated the patient's complaint, a problem, visible only in the R-R interval data plot.

FIGURE 13 is a diagram showing, by way of example, a diagnostic composite plot 90 for facilitating the diagnosis of bradycardia during sleep and a R-R interval pattern characteristic of sleep. Bradycardia refers to a resting heart rate of under 60 bpm. Bradycardia during sleep is often tempered with occasional spikes of rapid heart rate, which can be a secondary compensatory response to dreaming, snoring or sleep apnea. In a far field view 50 of R-R interval data, bradycardia manifests as the presence of a base line heart rate in the range of about 50 bpm 131, coupled with multiple spikes of dots 132 representing intermittent episodes of elevated heart rate. Such elevations in heart rate during a predominantly slower rate may be signs of a cardio-respiratory disorder. Still other applications of the diagnostic composite plot 80 are possible.

The diagnostic composite plots are a tool used by physicians as part of a continuum of cardiac care provisioning that begins with ECG monitoring, continues through diagnostic overread and finally, if medically appropriate, concludes with cardiac rhythm disorder treatment. Each of these steps involve different physical components that collaboratively allow physicians to acquire and visualize R-R interval and ECG data in a way that accurately depicts heart rate variability over time. Further, the data represented by the diagnostic composite plots can be provided to analysis software for cardiac event detection, and patient diagnosis or confirmation. FIGURE 14 is a block diagram showing a system 140 for facilitating diagnosis of cardiac rhythm disorders with the aid of a digital computer 150 in accordance with one embodiment. Each diagnostic composite plot 151 is based on ECG data 166 that has either been recorded by a conventional electrocardiograph (not shown) or retrieved or obtained from some other type of ECG monitoring and recording device. Following completion of the ECG monitoring, the ECG data is assembled into a diagnostic composite plot 151, which can be used by a physician to diagnosis and, if required, treat a cardiac rhythm disorder, or for other health care or related purposes.

Each diagnostic composite plot 151 is based on ECG data 166 that has been recorded over a period of observation, which can be for just a short term, such as during a clinic appointment, or over an extended time frame of months. ECG recordation and, in some cases, physiological monitoring can be provided through various types of ECG-capable monitoring ensembles, including a standardized 12-lead ECG setup (not shown), such as used for clinical ECG monitoring, a portable Holter-type ECG recorder for traditional ambulatory ECG monitoring (also not shown), or a wearable ambulatory ECG monitor.

One form of ambulatory ECG monitor 142 particularly suited to monitoring and recording ECG and physiological data employs an electrode patch 143 and a removable reusable (or single use) monitor recorder 144, such as described in commonly-assigned U.S. Patent No. 9,345,414, issued May 24, 2016, cited supra, and also, further described in detail below with respect to FIGURES 15-18. The electrode patch 143 and monitor recorder 144 are synergistically optimized to capture electrical signals from the propagation of low amplitude, relatively low frequency content cardiac action potentials, particularly the P-waves generated during atrial activation. The ECG monitor 142 sits centrally (in the midline) on the patient’s chest along the sternum 169 oriented top-to-bottom. The ECG monitor 142 interfaces to a pair of cutaneous electrodes (not shown) on the electrode patch 143 that are adhered to the patient’s skin along the sternal midline (or immediately to either side of the sternum 169). The ECG monitor 142 has a unique narrow "hourglass"-like shape that significantly improves the ability of the monitor to be comfortably worn by the patient 141 for an extended period of time and to cutaneously sense cardiac electric signals, particularly the P-wave (or atrial activity) and, to a lesser extent, the QRS interval signals in the ECG waveforms indicating ventricular activity.

The electrode patch 143 itself is shaped to conform to the contours of the patient’s chest approximately centered on the sternal midline. To counter the dislodgment due to compressional and torsional forces, a layer of non-irritating adhesive, such as hydrocolloid, is provided at least partially on the underside, or contact, surface of the electrode patch, but only on the electrode patch’s distal and proximal ends. To counter dislodgment due to tensile and torsional forces, a strain relief can be defined in the electrode patch’s flexible circuit using cutouts partially extending transversely from each opposite side of the flexible circuit and continuing longitudinally towards each other to define in ’S’-shaped pattern. In a further embodiment, the electrode patch 143 is made from a type of stretchable spunlace fabric. To counter patient bending motions and prevent disadhesion of the electrode patch 143, the outward-facing aspect of the backing, to which a (non-stretchable) flexible circuit is fixedly attached, stretches at a different rate than the backing’s skin-facing aspect, where a skin adhesive removably affixes the electrode patch 143 to the skin. Each of these components are distinctive and allow for comfortable and extended wear, especially by women, where breast mobility would otherwise interfere with ECG monitor use and comfort. Still other forms of ECG monitoring and recording assembles are possible.

When operated standalone, the monitor recorder 142 senses and records the patient’s ECG data 166 and physiological data (not shown) into a memory onboard the monitor recorder 144. The recorded data can be downloaded using a download station 147, which could be a dedicated download station 145 that permits the retrieval of stored ECG data 166 and physiological data, if applicable, execution of diagnostics on or programming of the monitor recorder 144, or performance of other functions. To facilitate physical connection with the download station 145, the monitor recorder 144 has a set of electrical contacts (not shown) that enable the monitor recorder 144 to physically interface to a set of terminals 148. In turn, the download station 145 can be operated through user controls 149 to execute a communications or data download program 146 ("Download") or similar program that interacts with the monitor recorder 144 via the physical interface to retrieve the stored ECG data 166. The download station 145 could alternatively be a server, personal computer, tablet or handheld computer, smart mobile device, or purpose-built device designed specific to the task of interfacing with a monitor recorder 144. Still other forms of download station 145 are possible. In a further embodiment, the ECG data 166 from the monitor recorder 144 can be offloaded wirelessly.

The ECG data 166 can be retrieved from the download station 145 using a control program 157 ("Ctl") or analogous application executing on a personal digital computer 156 or other connectable computing device, via a hard wired link 158, wireless link (not shown), or by physical transfer of storage media (not shown). The personal digital computer 156 may also execute middleware (not shown) that converts the ECG data 166 into a format suitable for use by a third-party post-monitoring analysis program. The personal digital computer 156 stores the ECG data 166 along with each patient’s electronic medical records (EMRs) 165 in the secure database 64, as further discussed infra. In a further embodiment, the download station 145 is able to directly interface with other devices over a computer communications network 155, which could be a combination of local area and wide area networks, including the Internet or another telecommunications network, over wired or wireless connections.

A client-server model can be employed for ECG data 166 analysis. In this model, a server 62 executes a patient management program 160 ("Mgt") or similar application that accesses the retrieved ECG data 166 and other information in the secure database 164 cataloged with each patient’s EMRs 165. The patients’ EMRs can be supplemented with other information (not shown), such as medical history, testing results, and so forth, which can be factored into automated diagnosis and treatment. The patient management program 160, or other trusted application, also maintains and safeguards the secure database 164 to limit access to patient EMRs 165 to only authorized parties for appropriate medical or other uses, such as mandated by state or federal law, such as under the Health Insurance Portability and Accountability Act (HIPAA) or per the European Union’s Data Protection Directive. Other schemes and safeguards to protect and maintain the integrity of patient EMRs 165 are possible.

In a further embodiment, the wearable monitor 142 can interoperate wirelessly with other wearable or implantable physiology monitors and activity sensors 152, such as activity trackers worn on the wrist or body, and with mobile devices 153, including smart watches and smartphones. Wearable or implantable physiology monitors and activity sensors 152 encompass a wide range of wirelessly interconnectable devices that measure or monitor a patient's physiological data, such as heart rate, temperature, blood pressure, respiratory rate, blood pressure, blood sugar (with or without an appropriate subcutaneous probe), oxygen saturation, minute ventilation, and so on; physical states, such as movement, sleep, footsteps, and the like; and performance, including calories burned or estimated blood glucose level. Frequently, wearable and implantable physiology monitors and activity sensors 152 are capable of wirelessly interfacing with mobile devices 153, particularly smart mobile devices, including so-called "smartphones" and "smart watches," as well as with personal computers and tablet or handheld computers, to download monitoring data either in real-time or in batches through an application ("App") or similar program.

Based on the ECG data 166, physicians can rely on the data as medically certifiable and are able to directly proceed with diagnosing cardiac rhythm disorders and determining the appropriate course of treatment for the patient 141, including undertaking further medical interventions as as appropriate. The ECG data 166 can be retrieved by a digital computer 150 over the network 155. A diagnostic composite plot 151 that includes multiple temporal points of reference and a plot of R-R interval data is then constructed based on the ECG data 166, as discussed in detail supra with reference to FIGURE 3, and displayed or, alternatively, printed, for use by a physician.

In a further embodiment, the server 159 executes a patient diagnosis program 161 ("Dx") or similar application that can evaluate the ECG data 166 to form a diagnosis of a cardiac rhythm disorder. The patient diagnosis program 161 compares and evaluates the ECG data 166 to a set of medical diagnostic criteria 167, from which a diagnostic overread 162 ("diagnosis") is ) generated. Each diagnostic overread 162 can include one or more diagnostic findings 168 that can be rated by degree of severity, such as with the automated diagnosis of atrial fibrillation. If at least one of the diagnostic findings 168 for a patient exceed a threshold level of tolerance, which may be tailored to a specific client, disease or medical condition group, or applied to a general patient population, in a still further embodiment, therapeutic treatment ("Therapy") to address diagnosed disorder findings can be generated and, optionally, programmed into a cardiac rhythm therapy delivery device, such as an IMD (not shown), including a pacemaker, implantable cardioverter defibrillator (ICD), or similar devices.

Facilitating diagnosis of cardiac rhythm disorders first requires the collection of ECG data from a patient. In one embodiment, the data can be collected via a wearable ambulatory ) ECG monitor having an electrode patch and a monitor recorder. The electrode patch is adhered to the patient's skin along the sternal midline or immediately to either side of the sternum to collect the data. A monitor recorder is then snapped into place on the electrode patch using an electro mechanical docking interface to initiate ECG monitoring. FIGURE 15 is a perspective view showing an ambulatory ECG monitor 212 having an extended wear electrode patch 215 with a monitor recorder 214 inserted. The body of the electrode patch 215 is preferably constructed using a flexible backing 220 formed as an elongated strip 221 of wrap knit or similar stretchable material about 145 mm long and 32 mm at the widest point with a narrow longitudinal mid-section 223 evenly tapering inward from both sides. However, other lengths and widths are possible. A pair of cut-outs 222 between the distal and proximal ends of the ) electrode patch 15 create a narrow longitudinal midsection 223 or "isthmus" and defines an elongated "hourglass"-like shape, when viewed from above, such as described in commonly-assigned U.S. Design Patent No. D744659, issued December 1, 2015. The upper part of the "hourglass" is sized to allow an electrically non-conductive receptacle 225, that sits on top of the outward-facing surface of the electrode patch 215, to be affixed to the electrode patch 215 with an ECG electrode placed underneath on the patient-facing underside, or contact, surface of the electrode patch 215; the upper part of the "hourglass" has a longer and wider profile (but still rounded and tapered to fit comfortably between the breasts) than the lower part of the "hourglass," which is sized primarily to allow just the placement of an ECG electrode of appropriate shape and surface area to record the P-wave and the QRS signals sufficiently given the inter-electrode spacing.

The electrode patch 215 incorporates features that significantly improve wearability, performance, and patient comfort throughout an extended monitoring period. The entire electrode patch 215 is lightweight in construction, which allows the patch to be resilient to disadhesing or falling off and, critically, to avoid creating distracting discomfort to the patient, ) even when the patient is asleep. In contrast, the weight of a heavy ECG monitor impedes patient mobility and will cause the monitor to constantly tug downwards and press on the patient's body that can generate skin inflammation with frequent adjustments by the patient needed to maintain comfort.

During every day wear, the electrode patch 215 is subjected to pushing, pulling, and torsional movements, including compressional and torsional forces when the patient bends forward, or tensile and torsional forces when the patient leans backwards. To counter these stress forces, the electrode patch 215 incorporates crimp and strain reliefs, such as described in commonly-assigned U.S. Patent No. 9,545,204, issued January 17, 2017. In a still further embodiment, tabs 224 can respectively extend an additional 8 mm to 12 mm beyond the distal ) and proximal ends of the flexible backing 220 to facilitate with adhering the electrode patch 215 to or removing the electrode patch 215 from the sternum 213. These tabs 224 preferably lack adhesive on the underside, or contact, surface of the electrode patch 215. Still other shapes, cut-outs and conformities to the electrode patch 215 are possible.

The monitor recorder 214 removably and reusably snaps into an electrically non-conductive receptacle 225 during use. The monitor recorder 214 contains electronic circuitry for recording and storing the patient's electrocardiography as sensed via a pair of ECG electrodes provided on the electrode patch 215. The non-conductive receptacle 225 is provided on the top surface of the flexible backing 220 with a retention catch 226 and tension clip 227 molded into the non-conductive receptacle 225 to conformably receive and securely hold the monitor ) recorder 214 in place.

The electrode patch 215 is generally intended for a single use and is meant to be replaced periodically throughout an extended period of monitoring. However, some types of monitoring may not extend over a period of time long enough to necessitate replacement of the electrode patch 215. In those situations, the monitor recorder 214 and electrode patch 215 can be combined into a single integral assembly.

The monitor recorder 214 includes a sealed housing that snaps into place in the non-conductive receptacle 225. FIGURE 16 is a perspective view showing the monitor recorder 214 of FIGURE 15. The sealed housing 250 of the monitor recorder 214 can have a rounded isosceles trapezoidal-like shape, when viewed from above, such as described in commonly-assigned U.S. Design Patent No. D717955, issued November 18, 2014. The edges 251 along the top and bottom surfaces are rounded for patient comfort. The sealed housing 250 is approximately 47 mm long, 23 mm wide at the widest point, and 7 mm high, excluding a patient-operable tactile-feedback button 255. However, other sizes of the sealed housing are possible.

) The sealed housing 250 can be molded out of polycarbonate, ABS, or an alloy of those two materials. The button 255 is waterproof and the button's top outer surface is molded silicon rubber or similar soft pliable material. A retention detent 253 and tension detent 254 are molded along the edges of the top surface of the housing 250 to respectively engage the retention catch 226 and the tension clip 227 molded into non-conductive receptacle 225. Other shapes, features, and conformities of the sealed housing 250 are possible.

The electrode patch 215 is intended to be disposable, while the monitor recorder 214 is designed for reuse and can be transferred to successive electrode patches 215 to ensure continuity of monitoring, if so desired. The monitor recorder 14 can be used only once, but single use effectively wastes the synergistic benefits provided by the combination of the ) disposable electrode patch and reusable monitor recorder. The placement of the wearable monitor 212 in a location at the sternal midline (or immediately to either side of the sternum) benefits long-term extended wear by removing the requirement that ECG electrodes be continually placed in the same spots on the skin throughout the monitoring period. Instead, the patient is free to place an electrode patch 215 anywhere within the general region of the sternum.

As a result, at any point during ECG monitoring, the patient's skin is able to recover from the wearing of an electrode patch 215, which increases patient comfort and satisfaction, while the monitor recorder 214 ensures ECG monitoring continuity with minimal effort. A monitor recorder 214 is merely unsnapped from a worn out electrode patch 215, the worn out electrode patch 215 is removed from the skin, a new electrode patch 215 is adhered to the skin, ) possibly in a new spot immediately adjacent to the earlier location, and the same monitor recorder 214 is snapped into the new electrode patch 215 to reinitiate and continue the ECG monitoring.

During use, the electrode patch 215 is first adhered to the skin in the sternal region. FIGURE 17 is a perspective view showing the extended wear electrode patch 215 of FIGURE 15 without a monitor recorder 214 inserted. A flexible circuit 232 is adhered to each end of the flexible backing 220. A distal circuit trace 233 from the distal end 230 of the flexible backing 220 and a proximal circuit trace (not shown) from the proximal end 231 of the flexible backing 220 electrically couple ECG electrodes (not shown) with a pair of electrical pads 234. In a further embodiment, the distal and proximal circuit traces are replaced with interlaced or sewn-in flexible wires. The electrical pads 234 are provided within a moisture-resistant seal 235 formed on the bottom surface of the non-conductive receptacle 225. When the monitor recorder 214 is securely received into the non-conductive receptacle 225, that is, snapped into place, the electrical pads 234 interface to electrical contacts (not shown) protruding from the bottom surface of the monitor recorder 214. The moisture-resistant seal 235 enables the monitor recorder 214 to be worn at all times, even during showering or other activities that could expose the monitor recorder 214 to moisture or adverse conditions.

In addition, a battery compartment 236 is formed on the bottom surface of the non-conductive receptacle 225. A pair of battery leads (not shown) from the battery compartment 236 to another pair of the electrical pads 234 electrically interface the battery to the monitor recorder 214. The battery contained within the battery compartment 235 is a direct current (DC) power cell and can be replaceable, rechargeable or disposable.

The monitor recorder 214 draws power externally from the battery provided in the non-conductive receptacle 225, thereby uniquely obviating the need for the monitor recorder 214 to carry a dedicated power source. FIGURE 18 is a bottom plan view of the monitor recorder 214 of FIGURE 15. A cavity 258 is formed on the bottom surface of the sealed housing 250 to accommodate the upward projection of the battery compartment 236 from the bottom surface of the non-conductive receptacle 225, when the monitor recorder 214 is secured in place on the non-conductive receptacle 225. A set of electrical contacts 256 protrude from the bottom surface of the sealed housing 250 and are arranged in alignment with the electrical pads 234 provided on the bottom surface of the non-conductive receptacle 225 to establish electrical connections between the electrode patch 215 and the monitor recorder 214. In addition, a seal coupling 257 circumferentially surrounds the set of electrical contacts 256 and securely mates with the moisture-resistant seal 235 formed on the bottom surface of the non-conductive receptacle 225. The battery contained within the battery compartment 236 can be replaceable, rechargeable or disposable. In a further embodiment, the ECG sensing circuitry of the monitor recorder 214 can be supplemented with additional sensors, including an SpO2 sensor, a blood pressure sensor, a temperature sensor, respiratory rate sensor, a glucose sensor, an air flow sensor, and a volumetric pressure sensor, which can be incorporated directly into the monitor recorder 214 or onto the non-conductive receptacle 225.

ECG data collected via the ambulatory ECG monitor, such as described above, or another monitor can be provided to a post-monitoring analysis program for cardiac event detection and patient diagnosis. However, prior to analysis by a program, noise can optionally be removed to refine the ECG data for more accurate detection and diagnosis results. For example, ECG data is often interspersed with events that occur while recording and represent device-related events, such as low battery warnings or reset events, which are not interesting to the analysis of the data. Detection and removal of noise not only increases data quality, but reduces an amount of data necessary for analysis, which can result in more accurate and quicker results. Additionally, areas of recorded data near a period of time when the patient presses the ) feedback button tends to look like noise, but is extremely important for consideration and analysis since the patient believes a cardiac event may be occurring.

Noise detection and removal can be performed prior to any processing of the ECG data or after some processing has occurred. FIGURE 19 is a flow diagram showing a method 270 for ECG data classification for facilitating diagnosis of cardiac rhythm disorders with the aid of a digital computer. Cutaneous action potentials of a patient are monitored and recorded (block 271) as ECG data over a set time period. Upon completion of the monitoring period, the ECG and any physiological data are downloaded or retrieved into a digital computer (block 272).

Some recorded signals within the collected ECG data are low amplitude and can be incorrectly detected as noise. Optionally, automatic gain control can be applied (block 273) to ) the recorded signals to place as many as the ECG signals as possible within a normal range, such as from 0.5 to 1.8 mV peak-to-peak, such as described in commonly-assigned U.S. Patent No. 9,345,414, issued May 24, 2016. Automatic gain control is generally provided through an AGC module that is implemented as part of a suite of post-processing modules, although automatic gain control could also be provided through standalone software or as part of other forms of ECG visualization and interpretation software. The automatic gain control can be run prior to or after noise detection. If prior, gain control is used to regularize the signal amplitude for noise detection; however, if run after, gain control is used to control the signal amplitude seen by the noise detection analysis software. Alternatively, the signal amplitude is controlled manually by a reading technician.

) The gain can be determined by defining a temporal window for automatic gain control. The temporal window has to be long enough to capture at least one heart beat; a five-second temporal window is used, although other durations are possible. The peak-to-peak voltage of each ECG data segment is computed and a single gain factor is applied to the entire signal such that the average signal falls in the center of the preferred range. Alternatively, other statistical values could be used to represent the correlation of the peak-to-peak voltages in the preferred range, either in lieu of or in addition to the average voltage, such as maximum or minimum observed voltage, mean voltage, and so on. Each ECG value in a segment is then processed in an iterative loop. The ECG values are dynamically gained. In one embodiment, the preferred range falls from about 2 mV to 10 mV, although other values could be chosen.

Subsequently, noise detection can optionally be performed (block 274) to refine the ECG data for analysis and use in patient diagnosis. The noise detection can include classifying segments of the ECG data as noise or valid data, as further described below with reference to FIGURES 19-25. A potentially-actionable cardiac event within a vector of the ECG data can be optionally identified and the ECG data during, prior to and after the event can be selected (block 275), as described supra with respect to FIGURE 3. In one example, the vector of ECG data can represent a 40-minute (or other duration) time span that is used in constructing the plot of R-R interval data, although other pre-event and post-event time spans are possible. The event could be identified with the assistance of a software package, such as Holter LX Analysis Software, licensed by NorthEast Monitoring, Inc., Maynard, MA; IntelliSpace Cardiovascular Image and Information management system, licensed Koninklijke Philips N.V., Amsterdam, Netherlands; MoMe System, licensed by InfoBionic, Lowell, MA; Pyramis ECG Management, licensed by Mortara Instrument Inc., Milwaukee, WI; ICS Clinical Suite, licensed by Spacelabs Healthcare Inc., Snoqualmie, WA; or a customized software package. Alternatively, the potentially-actionable cardiac event could be identified by a physician or technician during review of the ECG data.

Based on the vector of ECG data, a plot of R-R interval data is constructed (block 276) and can be displayed with both near field and far field contextual views of the ECG data, as a diagnostic composite plot, as described supra with respect to FIGURE 6. Both views are temporally keyed to an extended duration R-R interval data view that, in one embodiment, is scaled non-linearly to maximize the visual differentiation for frequently-occurring heart rate ranges, such that a single glance allows the physician to make a diagnosis. All three views are presented simultaneously, thereby allowing an interpreting physician to diagnose rhythm and the pre- and post-contextual events leading up to a cardiac rhythm of interest.

If not previously performed, noise detection can optionally be performed (block 277) on the R-R interval data to remove data segments classified as noise, as further described below with respect to FIGURE 20, to refine the data for further analysis. After, a diagnosis of a cardiac rhythm disorder can optionally be formed (block 278) based on findings made through interpretation of heart rate variability patterns in the diagnostic composite plot. For instance, the heart rate variability patterns in the diagnostic composite plot could be provided to a system that programmatically detects AF by virtue of looking for the classic Gaussian-type distribution on the "cloud" of heart rate variability formed in the plot of R-R interval data, which can be corroborated by the accompanying contextual ECG data. Finally, therapy to address diagnosed disorder findings can optionally be programmed into a cardiac rhythm therapy delivery device (step 279), such as an implantable medical device (IMD) (not shown), including a pacemaker, implantable cardioverter defibrillator (ICD), or similar devices.

A presence of noise in the ECG data can pose problems for cardiologists when diagnosing and caring for patients with possible cardiac arrhythmias. In contrast, identifying and removing noise by classifying segments of the data can greatly increase accuracy of identifying a valid cardiac event. FIGURE 20 is a flow diagram showing a method 290 for noise detection. Prior to classification of ECG data, an adaptive noise detector is trained (block 291). In one example, the adaptive noise detector can be implemented by a convolutional neural network that represents a processing device, such as an algorithm executed by a computer processor or actual hardware. Other types of systems are possible.

During training, segments of the ECG data are annotated with a classification of noise or valid data by software analysis or human review. Training is further described in detail below with respect to FIGURE 21. Once trained, further ECG data collected from a patient via an ambulatory ECG monitor, such as the monitor described above with respect to FIGURES 15-18, is segmented and provided (block 292) to the trained adaptive noise detector for classification (block 293). However, data from other types of ECG monitors can be used.

Classification of the ECG data segments includes assigning a designation of valid data or noise to each segment. Subsequently, the ECG data segments that correspond with a time frame during which a patient feedback button on the ECG ambulatory monitor is pressed are identified. Those segments classified as noise and that overlap with the timeframe are trimmed (block 294) to remove the data corresponding with the button press. Defining the time frame is further described in detail below with reference to FIGURE 23. Finally, all segments classified as noise can be removed from the ECG data prior to further processing. In a further embodiment, the noise data segments can remain with the ECG data; however, during review by analysis software, those segments classified as noise can be ignored.

Accurate classification of the ECG data segments is dependent on training accuracy. FIGURE 21 is a flow diagram showing, by way of example, a method 300 for training an adaptive noise detector. ECG training data is obtained (block 301) as data files from ambulatory ECG monitors associated with a group of patients. The patients can be selected randomly or identified based on patient condition. In one embodiment, between 200 and 250 files can be collected from different ECG ambulatory monitors and used as training data. The ambulatory monitors used to collect the ECG data can include the monitor described above with respect to FIGURES 15-18, as well as other types of monitors. The data file collected from each ECG ambulatory monitor can each include up to or more than 64MB of data.

The training data is divided (block 302) into segments, and regions of the training data are annotated (block 303) with markers for valid data or noise by software analysis, human review, or a combination of software analysis and human review. Specifically, each file from one of the ambulatory ECG monitors is divided into segments. For example, the ECG data from a single file can be divided into over 1500 segments, such that each segment includes between 9-10 seconds of data. However, other numbers of segments and data times represented by each segment are possible, such as segments of data between 2 and 12 seconds. The data can be divided into contiguous segments or alternatively, into overlapping segments. In one embodiment, a neural network views only one segment at a time for detection and thus, each segment should include enough data for a determination of noise. The periodicity of heart activity is an important indicator of non-noise so each segment should include at least a few beats. However, the larger the segment becomes the more good signal will be mis-marked as noise on either side of a real noise identification.

Optionally, a portion of the data at the beginning of each training segment can be removed (block 304) since such data often includes or resembles noise, which could lead to misclassification of the data in that segment. The training data segments are then provided (block 305) to the adaptive noise detector. In one embodiment, the segments of the training data are each provided and run through the adaptive noise detector once. In a further embodiment, the training segments are run multiple times.

After the training data has been run through the adaptive noise detector, testing can be performed (block 306) to determine classification accuracy of the adaptive noise detector. During testing, a set of testing data, such as collected from ECG ambulatory monitors, is run through the trained adaptive noise detector, which classifies the testing data. Additionally, the testing data is annotated manually or via a different program with classifications for noise or valid data. A comparison is made between the annotation of the data and the results of the adaptive noise detector. A number of samples correctly classified is determined and an average accuracy of the testing data is determined. In one embodiment, the average accuracy can be determined and reported after every n number of batches of ECG data. For example, n can represent 100 batches; however, other numbers of batches are possible. The batches can be determined based on an amount of ECG data, a number of ECG ambulatory monitors providing the data, as well as by other metrics. In one embodiment, the batch should be small enough to fit in the memory available, but large enough to amortize startup time. An accuracy threshold can be applied to the average accuracy values and if an average accuracy value or a predetermined number of average accuracy values are below the threshold, further training can be performed (block 307) to increase the accuracy of the adaptive noise detector.

Once the adaptive noise detector is accurately trained, further ECG data is collected and provided to the detector for classification. FIGURE 22 is a flow diagram showing, by way of example, a method 310 for classification of ECG data. The collected ECG data is segmented into blocks, such as described above with respect to the training data of FIGURE 21. The ECG data segments are then received (block 311) by the adaptive noise detector, which can be implemented by a convolutional neural network utilizing, for example, a one dimensional formulation for use with ECG data. Additionally, the adaptive noise detector can include hidden layers for performing the classification. In the example described below, two convolutional or pooling hidden layers, and two fully-connected hidden layers are utilized. However, other number of layers are possible.

During the first convolution layer, ECG trace features are identified (block 312) using, for example, sliding filters. Examples of ECG trace features can include two R waves with three P waves located between the R waves and R wave spikes, as well as other types of features that are indicative of a cardiac event, such as an arrhythmia. The features can be automatically learned during the training process and take any form that is learned to be associated with noise or the ECG. In one embodiment, filters for at least 32 features are run against the ECG data. During the second convolution layer, repeating patterns of the features are identified (block 313), including, for example, identifying high numbers of successive R wave spikes.

Next, the data obtained from the second convolution layer is provided to a first fully connected cross-connection layer, which builds (block 314) a matrix with the repeating features representing the columns and matrix multipliers representing rows. An intersection value for each combination of the repeating features and matrix multipliers are listed in the matrix as cross connection weights. Specifically, the intersection value can be determined by multiplying each repeating feature value with a matrix multiplier and scaling the product by a predetermined factor. However, other methods for determining the intersection values are possible.

The second fully connected cross-connection layer utilizes the cross-connection weights from the first fully connected cross-connection layer and multiplies the cross-connection weights by further weights to calculate (block 315) final cross-connection values for each ECG data segment. The final cross-connection values include a noise classification value and a valid data classification value. The noise classification value and the valid data classification value indicate a likelihood that the data segment, respectively, includes noise or does not include noise.

Based on the final cross-connection values, a determination is made as to whether the noise classification value for each data segment exceeds (block 316) the valid data classification value, and if so, the noise classification is assigned (block 317) to that data segment. Otherwise, if the noise classification value does not exceed (block 316) the valid data classification value, a valid data classification is assigned (block 318) to that data segment. As described above, with respect to FIGURE 20, the segments classified as noise are removed from the ECG data and the remaining valid data segments are provided for further analysis and patient diagnosis.

However, prior to such analysis, patient identified cardiac events are identified and considered. Patient identified cardiac events are important for use in diagnosis since the patient generally provides feedback when she feels uncomfortable, ill, or is experiencing symptoms resembling a cardiac event. Such feedback is obtained when the patient applies physical pressure to the feedback button located on the monitor recorder of the ECG ambulatory monitor, as shown in FIGURE 16.

Often times, though, the physical pressure, from the patient, causes the button press to appear as noise in the recorded data. Due to the importance of the data prior to, during, and after the button press, if the data collected at the time of the button press is marked as noise, some or all of the related data will be removed from further analysis. Thus, to maintain data related to the button press, segments with data that overlap a button press event and that are classified as noise are trimmed to remove the data related to the button from being classified as noise.

The trimming can be performed based on defined button press windows. FIGURE 23 is a flow diagram showing, by way of example, a method 320 for trimming data segments classified as noise. Within the ECG data for one or more ECG ambulatory monitors, presses of the patient feedback button are identified (block 321) and marked. For each button press event, a button window is generated (block 322) by identifying a predetermined time before and after each button press event and designating the span of time as a button window. In one example, the predetermined times before and after a button press event can be the same, such as five minutes for a 10 minute window, or a different amount of time. However, in a further embodiment, each of the predetermined time before and after can differ.

The data segments classified as noise are identified (block 323) and compared with the button press windows to further identify (block 324) those noise data segments that overlap a button press window. The overlapping noise data segments are trimmed (block 325) to align with a start or end of the button press window, such that the noise data segment includes no ECG data within the button press window. The data removed from the noise data segment is assigned a valid data classification to ensure the data is later reviewed. Further, a noise classification assigned to any data segment that is fully encompassed by the button window is removed so that the data can be considered. Finally, the data segments classified as noise are removed from the ECG data, leaving only the data segments classified as valid and the data associated with the button presses for further analysis and use in patient diagnosis.

The button press windows can be displayed in a diagnostic composite plot for analysis by a medical professional. FIGURE 24 is a diagram showing, by way of example, a diagnostic composite plot 340 showing data from a button press window. The diagnostic composite plot 340, as described above with respect to FIGURE 6, includes an ECG plot presenting a near field (short duration) view 51, an ECG plot presenting an intermediate field (medium duration) view 52, and an R-R interval data plot presenting a far field (extended duration) view 53. A temporal point of reference, such as a button press event, is identified and centered on the x-axis in all three views. The button press event is associated with a button press window 341, which is displayed on the intermediate field view. In a further embodiment, all button presses in the ECG data recording can be displayed. FIGURE 25 is a diagram showing, by way of example, a summary graph 350, showing instances of button presses. A summary graph 350 maps all ECG data recorded by the ECG ambulatory monitor over a period up to seven days. The data is displayed and button presses can be identified via an icon 351. Identifying all instances of button presses over a recording period can be useful to identify patterns of symptoms experienced by the patient, which can assist in making a diagnosis.

Returning to the composite plot 340 of FIGURE 24, the placement of a single button press window in the middle of the x-axes on all three views enables the ECG data to be temporally keyed to the R-R interval data appearing in the center of the R-R interval data view 53, with a near field view 51 of an ECG displayed at normal (paper-based) recording speed and an intermediate field view 52 that presents the ECG data occurring before and after the center. As a result, the near field view 51 provides the ECG data corresponding to the R-R interval data at the center (or another location), while the intermediate field view 52 enables presentation of the broader ECG data context going beyond the borders of the near field view 51. In a further embodiment, the center can be slidably adjusted backwards and forwards in time, with the near field view 51 and the far field view 52 of the ECG data automatically adjusting accordingly to stay in context with the R-R interval data view 51. In a still further embodiment, multiple temporal points of reference can be identified with each temporal point of reference being optionally accompanied by one or more dedicated sets of ECG data views.

The intermediate field view 52 also displays data segments 342, dn, which each represent a grouping of ECG data that has been classified as valid or noise. In one embodiment, the classification for each data segment 342 can also be displayed. The segments 342 that are assigned a noise classification are compared with the button press window 341. Those noise data segments that overlap with the button press window 341 are trimmed. For example, data segment d8 overlaps with the button press window 341. Accordingly, the overlapping data 343 at the beginning of the button press window is removed from the data segment that is classified as noise, thus trimming the noise data segment. The trimmed data can be added to the next consecutive segment or form a new segment.

Subsequently, in one embodiment, a noise threshold can be applied to the data segments classified as noise to prevent shorts bursts of noise within one such segment from requiring all data in that segment to be removed, since some types of arrhythmias can resemble noise. If the data identified as noise within the noise segment occurs over a period that meets or exceeds the noise threshold, then that noise data segment is removed from the ECG data prior to analysis. However, if the data identified as noise within the noise data segment occurs over a period less than the noise threshold, the noise classification can be removed and the data remains with the ECG data. In one embodiment, each segment is assigned a noise determination of yes or no, with a yes determination indicating noise is present and a no determination indicating no noise is present in the segment. The threshold can be applied to one or more segments and can, in one instance, be equal to the length of a predetermined number of segments. For example, the threshold can be set to 20 seconds based on two 10 second segments of data and can be applied to multiple consecutive segments of data.

Finally, the data segments classified as noise are removed from the ECG data, leaving only the valid data segments and data associated with the button press windows for further analysis. In one embodiment, such data is provided for software analysis or human review for patient diagnosis, including identification of one or more cardiac events.

## Claims

1. A system (140) for ECG data classification for use in facilitating diagnosis of cardiac rhythm disorders with the aid of a digital computer, comprising:

   a database (164) to store ECG data (166) obtained via an electrocardiography monitor (212) comprising a patient button (255); and
   a server comprising a central processing unit, memory, an input port to receive the ECG data (166) from the database (164), and an output port, wherein the central processing unit is configured to:

identify within the ECG data (166) at least one press of the patient button (255);
define (322) within the ECG data (166) a button window comprising a segment of data prior to and after the press of the patient button (255);

divide the ECG data (166) into blocks;
apply noise detection analysis to the blocks of ECG data (166);
assign a classification comprising one of noise and valid data to each ECG data block based on the noise detection analysis via a trained adaptive noise detector;
identify (324) at least one block of ECG data (166) assigned the noise classification that overlaps the button window;
change a designation of the block of ECG data from the noise classification to the valid classification to ensure the ECG data in that block is later reviewed;
trim (325) the at least one block of ECG data (166) to align with one of the start and end of the button window;
maintain the trimmed block of ECG data as valid data; and

remove (326) from the ECG data (166) the blocks and the trimmed blocks assigned with the noise classification.

**2.** A system (140) in accordance with Claim 1, wherein the central processing unit performs the noise detection analysis by:

identifying (312) features;
identifying (313) repeating patterns of the features;
generating (314) a matrix with the identified features and patterns;
generating at least one matrix with weights for the identified features and patterns; and
assigning (318, 319) a value for each of the noise classification and the valid classification to each block based on the weight matrix.

**3.** A system (140) in accordance with Claim 2, wherein the central processing unit performs one or more of the following:

determining the features by applying (312) one or more sliding filters to the ECG data (166);
selecting (316) for one such block the classification assigned the highest value; and
performing an analysis of the ECG data (166) after the blocks assigned the noise classification have been removed and identifying at least one cardiac event based on the analysis.

**4.** A system (140) in accordance with Claim 3, wherein the central processing unit applies a time threshold to a period of identified noise in each of one or more blocks and assigns a noise classification to one such block when the noise classification has a higher value than the valid classification and the period of identified noise exceeds the time threshold.

**5.** A system (140) in accordance with Claim 1, wherein the central processing unit removes from each block of ECG data (166) a predetermined amount of data from a beginning of the block from consideration during the noise detection analysis.

**6.** A system (140) in accordance with Claim 1, wherein the electrocardiography monitor (212) comprises:
an extended wear electrode patch (215) comprising:

an elongated strip (221);
a pair of electrocardiographic electrodes conductively exposed on a contact surface of each end of the elongated strip (221), wherein one of the electrocardiographic electrodes is adhered to a region overlying the Xiphoid process on a patient's chest and an other of the electrocardiographic electrodes is adhered to the region near the manubrium on the patient's chest oriented centrally (in the midline) along the sternum upwards from the one electrocardiographic electrode;
a non-conductive receptacle (225) adhered to an outward-facing surface of the elongated strip (221) and comprising a plurality of electrical pads (234);
a battery electrically interfaced to a pair of the electrical pads (234) on the non-conductive receptacle (225); and
a circuit (232, 233) affixed on each end of the elongated strip (221).

**7.** A system (140) in accordance with Claim 6, wherein the electrocardiography monitor (212) further comprises:
a monitor recorder (214) having a sealed housing (250) adapted to be removably secured into the non-conductive receptacle (225) of the electrode patch, wherein the sealed housing (250) comprises a set of electrical contacts (256) that protrude from a bottom surface and that correspond with further electrical pads (234) on the non-conductive receptacle (225) and comprising:

a micro-controller operable to sense electrocardiographic signals through the electrocardiographic electrodes via the pair of the electrical pads (234); and
a flash memory electrically interfaced with the micro-controller and operable to store samples of the electrocardiographic signals.

**8.** A method (270) for ECG data classification for use in facilitating diagnosis of cardiac rhythm disorders with the aid of a digital computer, comprising:

obtaining ECG data (166) via an electrocardiography monitor (212) comprising a patient button (255);
identifying within the ECG data (166) at least one press of the patient button (255);
defining (322) within the ECG data (166) a button window comprising a segment of data prior to and after the press of the patient button (255);
dividing the ECG data (166) into blocks;
applying noise detection analysis to the blocks of ECG data (166);
assigning (318, 319) a classification comprising one of noise and valid data to each ECG data block based on the noise detection analysis via a trained adaptive noise detector;
identifying (324) at least one block of ECG data (166) assigned the noise classification that overlaps the button window;
changing a designation of the block of ECG data from the noise classification to the valid classification to ensure the ECG data in that block is later reviewed;
trimming (325) the at least one block of ECG data (166) to align with one of the start and end of the button window;
maintaining the trimmed block of ECG data as valid data; and
removing (326) from the ECG data (166) the blocks and the trimmed blocks assigned with the noise classification.

**9.** A method (270) in accordance with Claim 8, further comprising:

performing an analysis of the ECG data (166) after the blocks assigned the noise classification have been removed; and
identifying at least one cardiac event based on the analysis.

**10.** A method (270) in accordance with Claim 8, further comprising:
performing the noise detection analysis, comprising:

identifying (312) features;
identifying (313) repeating patterns of the features;
generating (314) a matrix with the identified features and patterns;
generating at least one matrix with weights for the identified features and patterns; and
assigning (318, 319) a value for each of the noise classification and the valid classification to each block based on the weight matrix.

**11.** A method (270) in accordance with Claim 10, further comprising at least one of:

determining (312) the features by applying one or more sliding filters to the ECG data (166); and
selecting (316) for one such block the classification assigned the highest value.

**12.** A method (270) in accordance with Claim 11, further comprising:

applying a time threshold to a period of identified noise in each of one or more blocks; and
assigning a noise classification to one such block when the noise classification has a higher value than the valid classification and the period of identified noise exceeds the time threshold.

**13.** A method (270) in accordance with Claim 8, further comprising:

from each block of ECG data (166), removing a predetermined amount of data from a beginning of the block from consideration during the noise detection analysis.

**14.** A method (270) in accordance with Claim 8, wherein the electrocardiography monitor (212) comprises: an extended wear electrode patch (215) comprising:

an elongated strip (221);
a pair of electrocardiographic electrodes conductively exposed on a contact surface of each end of the elongated strip (221), wherein one of the electrocardiographic electrodes is adhered to a region overlying the Xiphoid process on a patient's chest and an other of the electrocardiographic electrodes is adhered to the region near the manubrium on the patient's chest oriented centrally (in the midline) along the sternum upwards from the one electrocardiographic electrode;
a non-conductive receptacle (225) adhered to an outward-facing surface of the elongated strip (221) and comprising a plurality of electrical pads (234);
a battery electrically interfaced to a pair of the electrical pads (234) on the non-conductive receptacle (225); and
a circuit (232, 233) affixed on each end of the elongated strip (221)

**15.** A method (270) in accordance with Claim 14, wherein the electrocardiography monitor (212) further comprises: a monitor recorder (214) having a sealed housing (250) adapted to be removably secured into the non-conductive receptacle (225) of the electrode patch (215), wherein the sealed housing (250) comprises a set of electrical contacts (256) that protrude from a bottom surface and that correspond with further electrical pads (234) on the non-conductive receptacle (225) and comprising:

a micro-controller operable to sense electrocardiographic signals through the electrocardiographic electrodes via the pair of the electrical pads (234); and
a flash memory electrically interfaced with the micro-controller and operable to store samples of the electrocardiographic signals.

## Patentansprüche

**1.** Ein System (140) zur Klassifizierung von EKG-Daten zur Verwendung bei der Erleichterung der Diagnose von Herzrhythmusstörungen mit Hilfe eines digitalen Computers, das Folgendes beinhaltet:

eine Datenbank (164) zum Speichern von EKG-Daten (166), die über einen eine Patiententaste (255) beinhaltenden Elektrokardiographiemonitor (212) erhalten wurden; und
einen Server, der eine Zentraleinheit, einen Speicher, einen Eingangsanschluss zum Empfangen der EKG-Daten (166) von der Datenbank (164) und einen Ausgangsanschluss beinhaltet, wobei die Zentraleinheit für Folgendes konfiguriert ist:

Identifizieren, innerhalb der EKG-Daten (166), von mindestens einem Drücken der Patiententaste (255);
Definieren (322) innerhalb der EKG-Daten (166) eines Tastenfensters, das ein Datensegment vor und nach dem Drücken der Patiententaste (255) beinhaltet; Unterteilen der EKG-Daten (166) in Blöcke;
Anwenden von Rauschdetektionsanalyse auf die Blöcke der EKG-Daten (166);
Zuweisen einer Klassifizierung, die entweder Rauschen oder gültige Daten beinhaltet, zu jedem EKG-Datenblock basierend auf der Rauschdetektionsanalyse über einen trainierten adaptiven Rauschdetektor;
Identifizieren (324) mindestens eines Blocks von EKG-Daten (166), dem die Rauschklassifizierung zugewiesen wurde, der das Tastenfenster überlappt;
Ändern einer Bezeichnung des EKG-Datenblocks von der Rauschklassifizierung in die gültige Klassifizierung, um sicherzustellen, dass die EKG-Daten in diesem Block später überprüft werden;
Trimmen (325) des mindestens einen Blocks von EKG-Daten (166), um ihn nach dem Anfang oder dem Ende des Tastenfensters auszurichten;
Beibehalten des getrimmten Blocks von EKG-Daten als gültige Daten; und
Entfernen (326), aus den EKG-Daten (166), der Blöcke und der getrimmten Blöcke, die mit der Rauschklassifizierung zugewiesen wurden.

**2.** System (140) gemäß Anspruch 1, wobei die Zentraleinheit die Rauschdetektionsanalyse durch Folgendes durchführt:

Identifizieren (312) von Merkmalen;
Identifizieren (313) von sich wiederholenden Mustern der Merkmale;
Erzeugen (314) einer Matrix mit den identifizierten Merkmalen und Mustern;
Erzeugen mindestens einer Matrix mit Gewichten für die identifizierten Merkmale und Muster; und
Zuweisen (318, 319) eines Wertes für jede von der Rauschklassifizierung und der gültigen Klassifizierung zu jedem Block basieren auf der Gewichtsmatrix.

**3.** System (140) gemäß Anspruch 2, wobei die Zentraleinheit eines oder mehrere von Folgendem durchführt:

Bestimmen der Merkmale durch Anwenden (312) eines oder mehrerer Gleitfilter auf die EKG-Daten (166);
Auswählen (316) für einen solchen Block der Klassifizierung, welcher der höchste Wert zugewiesen wurde; und
Durchführen einer Analyse der EKG-Daten (166), nachdem die Blöcke, denen die Rauschklassifizierung zugewiesen wurde, entfernt wurden, und Identifizieren mindestens eines kardialen Ereignisses basierend auf der Analyse.

**4.** System (140) gemäß Anspruch 3, wobei die Zentraleinheit einen Zeitschwellenwert auf eine Periode identifizierten Rauschens in jedem von einem oder mehreren Blöcken anwendet und einem solchen Block eine Rauschklassifizierung zuweist, wenn die Rauschklassifizierung einen höheren Wert als die gültige Klassifizierung aufweist und die Periode identifizierten Rauschens den Zeitschwellenwert überschreitet.

**5.** System (140) gemäß Anspruch 1, wobei die Zentraleinheit aus jedem Block von EKG-Daten (166) während der Rauschdetektionsanalyse eine vorbestimmte Menge von Daten von einem Anfang des Blocks aus der Betrachtung entfernt.

**6.** System (140) gemäß Anspruch 1, wobei der Elektrokardiographiemonitor (212) Folgendes beinhaltet:
ein Elektrodenpflaster (215) mit verlängerter Tragedauer, das Folgendes beinhaltet:

einen länglichen Streifen (221);
ein Paar elektrokardiographischer Elektroden, die leitend auf einer Kontaktfläche jedes Endes des länglichen Streifens (221) freigelegt sind, wobei eine der elektrokardiographischen Elektroden an einen Bereich über dem Schwertfortsatz auf der Brust eines Patienten geklebt ist und eine andere der elektrokardiographischen Elektroden an den Bereich in der Nähe des Manubriums auf der Brust des Patienten geklebt ist, der zentral (in der Mittellinie) entlang des Brustbeins von der einen elektrokardiographischen Elektrode aufwärts ausgerichtet ist;
einen nicht leitenden Behälter (225), der an einer nach außen weisenden Oberfläche des länglichen Streifens (221) haftet und eine Vielzahl von elektrischen Pads (234) beinhaltet;
eine Batterie, die elektrisch mit einem Paar der elektrischen Pads (234) auf dem nicht leitenden Behälter (225) verbunden ist; und
eine Schaltung (232, 233), die an jedem Ende des länglichen Streifens (221) angebracht ist.

**7.** System (140) gemäß Anspruch 6, wobei der Elektrokardiographiemonitor (212) ferner Folgendes beinhaltet:

ein Überwachungsaufzeichnungsgerät (214), das ein abgedichtetes Gehäuse (250) aufweist, das angepasst ist, um abnehmbar in dem nicht leitenden Behälter (225) des Elektrodenpflasters befestigt zu werden, wobei das abgedichtete Gehäuse (250) einen Satz elektrischer Kontakte (256) beinhaltet, die von einer Bodenfläche vorstehen und
mit weiteren elektrischen Pads (234) an dem nicht leitenden Behälter (225) korrespondieren, und das Folgendes beinhaltet:

einen Mikrocontroller, der betreibbar ist, um elektrokardiographische Signale durch die elektrokardiographischen Elektroden über das Paar der elektrischen Pads (234) zu erfassen; und
einen Flash-Speicher, der elektrisch mit dem Mikrocontroller verbunden ist und zum Speichern von Proben der elektrokardiographischen Signale verwendet werden kann.

**8.** Ein Verfahren (270) zur Klassifizierung von EKG-Daten zur Verwendung bei der Erleichterung der Diagnose von Herzrhythmusstörungen mit Hilfe eines digitalen Computers, das Folgendes beinhaltet:

Erhalten von EKG-Daten (166) über einen eine Patiententaste (255) beinhaltenden Elektrokardiographiemonitor (212);

Identifizieren, innerhalb der EKG-Daten (166), von mindestens einem Drücken der Patiententaste (255);
Definieren (322) innerhalb der EKG-Daten (166) eines Tastenfensters, das ein Datensegment vor und nach dem Drücken der Patiententaste (255) beinhaltet;
Unterteilen der EKG-Daten (166) in Blöcke;
Anwenden von Rauschdetektionsanalyse auf die Blöcke der EKG-Daten (166);
Zuweisen (318, 319) einer Klassifizierung, die entweder Rauschen oder gültige Daten beinhaltet, zu jedem EKG-Datenblock basierend auf der Rauschdetektionsanalyse über einen trainierten adaptiven Rauschdetektor;
Identifizieren (324) mindestens eines Blocks von EKG-Daten (166), dem die Rauschklassifizierung zugewiesen wurde, der das Tastenfenster überlappt;
Ändern einer Bezeichnung des EKG-Datenblocks von der Rauschklassifizierung in die gültige Klassifizierung, um sicherzustellen, dass die EKG-Daten in diesem Block später überprüft werden;
Trimmen (325) des mindestens einen Blocks von EKG-Daten (166), um ihn nach dem Anfang oder dem Ende des Tastenfensters auszurichten;
Beibehalten des getrimmten Blocks von EKG-Daten als gültige Daten; und
Entfernen (326), aus den EKG-Daten (166), der Blöcke und der getrimmten Blöcke, die mit der Rauschklassifizierung zugewiesen wurden.

**9.** Verfahren (270) gemäß Anspruch 8, das ferner Folgendes beinhaltet:

Durchführen einer Analyse der EKG-Daten (166), nachdem die Blöcke, denen die Rauschklassifizierung zugewiesen wurde, entfernt wurden; und
Identifizieren mindestens eines kardialen Ereignisses basierend auf der Analyse.

**10.** Verfahren (270) gemäß Anspruch 8, das ferner Folgendes beinhaltet:
Durchführen der Rauschdetektionsanalyse, die Folgendes beinhaltet:

Identifizieren (312) von Merkmalen;
Identifizieren (313) von sich wiederholenden Mustern der Merkmale;
Erzeugen (314) einer Matrix mit den identifizierten Merkmalen und Mustern;
Erzeugen mindestens einer Matrix mit Gewichten für die identifizierten Merkmale und Muster; und
Zuweisen (318, 319) eines Wertes für die Rauschklassifizierung und die gültige Klassifizierung zu jedem Block basieren auf der Gewichtsmatrix.

**11.** Verfahren (270) gemäß Anspruch 10, das ferner mindestens eines von Folgendem beinhaltet:

Bestimmen (312) der Merkmale durch Anwenden eines oder mehrerer Gleitfilter auf die EKG-Daten (166); und
Auswählen (316) für einen solchen Block der Klassifizierung, welcher der höchste Wert zugewiesen wurde.

**12.** Verfahren (270) gemäß Anspruch 11, das ferner Folgendes beinhaltet:

Anwenden eines Zeitschwellenwerts auf eine Periode identifizierten Rauschens in jedem von einem oder mehreren Blöcken; und
Zuweisen einer Rauschklassifizierung zu einem solchen Block, wenn die Rauschklassifizierung einen höheren Wert als die gültige Klassifizierung aufweist und die Periode identifizierten Rauschens den Zeitschwellenwert überschreitet.

**13.** Verfahren (270) gemäß Anspruch 8, das ferner Folgendes beinhaltet:
aus jedem Block von EKG-Daten (166), Entfernen einer vorbestimmten Menge von Daten von einem Anfang des Blocks aus der Betrachtung während der Rauschdetektionsanalyse.

**14.** Verfahren (270) gemäß Anspruch 8, wobei der Elektrokardiographiemonitor (212 Folgendes beinhaltet:
ein Elektrodenpflaster mit verlängerter Tragedauer (215), das Folgendes beinhaltet:

einen länglichen Streifen (221);
ein Paar elektrokardiographischer Elektroden, die leitend auf einer Kontaktfläche jedes Endes des länglichen Streifens (221) freigelegt sind, wobei eine der elektrokardiographischen Elektroden an einen Bereich über dem Schwertfortsatz auf der Brust eines Patienten geklebt ist und eine andere der elektrokardiographischen Elektroden an den Bereich in der Nähe des Manubriums auf der Brust des Patienten geklebt ist, der zentral (in der Mittellinie) entlang des Brustbeins von der einen elektrokardiographischen Elektrode aufwärts ausgerichtet ist;
einen nicht leitenden Behälter (225), der an einer nach außen weisenden Oberfläche des länglichen Streifens (221) haftet und eine Vielzahl von elektrischen Pads (234) beinhaltet;
eine Batterie, die elektrisch mit einem Paar der elektrischen Pads (234) auf dem nicht leitenden Behälter (225) verbunden ist; und
eine Schaltung (232, 233), die an jedem Ende des länglichen Streifens (221) angebracht ist.

**15.** Verfahren (270) gemäß Anspruch 14, wobei der Elektrokardiographiemonitor (212) ferner Folgendes beinhaltet: ein Überwachungsaufzeichnungsgerät (214), das ein abgedichtetes Gehäuse (250) aufweist, das angepasst ist, um abnehmbar in dem nicht leitenden Behälter (225) des Elektrodenpflasters (215) befestigt zu werden, wobei das abgedichtete Gehäuse (250) einen Satz elektrischer Kontakte (256) beinhaltet, die von einer Bodenfläche vorstehen und mit weiteren elektrischen Pads (234) an dem nicht leitenden Behälter (225) korrespondieren, und das Folgendes beinhaltet:

einen Mikrocontroller, der betreibbar ist, um elektrokardiographische Signale durch die elektrokardiographischen Elektroden über das Paar der elektrischen Pads (234) zu erfassen; und
einen Flash-Speicher, der elektrisch mit dem Mikrocontroller verbunden ist und zum Speichern von Proben der elektrokardiographischen Signale verwendet werden kann.

**Revendications**

**1.** Un système (140) destiné à la classification de données ECG pour son utilisation dans la facilitation de diagnostics de troubles du rythme cardiaque à l'aide d'un ordinateur numérique, comprenant :

une base de données (164) pour stocker des données ECG (166) obtenues par l'intermédiaire d'un dispositif de surveillance d'électrocardiographie (212) comprenant un bouton pour patient (255) ; et
un serveur comprenant une unité centrale de traitement, une mémoire, un port d'entrée pour recevoir les données ECG (166) en provenance de la base de données (164), et
un port de sortie, où l'unité centrale de traitement est configurée pour :

identifier au sein des données ECG (166) au moins une pression du bouton pour patient (255) ;
définir (322) au sein des données ECG (166) un créneau de bouton comprenant un segment de données avant et après la pression du bouton pour patient (255) ; répartir les données ECG (166) en blocs ;
appliquer une analyse de détection de bruit aux blocs de données ECG (166) ;
attribuer une classification comprenant un élément parmi du bruit et des données valides à chaque bloc de données ECG sur la base de l'analyse de détection de bruit par l'intermédiaire d'un détecteur de bruit adaptatif entraîné ;
identifier (324) au moins un bloc de données ECG (166) auquel a été attribuée la classification de bruit qui chevauche le créneau de bouton ;
modifier une désignation du bloc de données ECG de la classification de bruit en la classification valide afin de s'assurer que les données ECG dans ce bloc soient revues par la suite ;
tronquer (325) l'au moins un bloc de données ECG (166) afin qu'il s'aligne sur un élément parmi le début et la fin du créneau de bouton ;
maintenir le bloc de données ECG tronqué comme données valides ; et
éliminer (326) des données ECG (166) les blocs et les blocs tronqués auxquels la classification de bruit a été attribuée.

**2.** Un système (140) conformément à la revendication 1, où l'unité centrale de traitement réalise l'analyse de détection de bruit par :

identification (312) de caractéristiques ;
identification (313) de motifs répétitifs des caractéristiques ;
génération (314) d'une matrice avec les caractéristiques et motifs identifiés ;
génération d'au moins une matrice avec des poids pour les caractéristiques et motifs identifiés ; et
attribution (318, 319) d'une valeur pour chacune parmi la classification de bruit et la classification valide à chaque bloc sur la base de la matrice de poids.

3. Un système (140) conformément à la revendication 2, où l'unité centrale de traitement réalise une ou plusieurs des actions suivantes :

la détermination des caractéristiques par application (312) d'un ou de plusieurs filtres glissants aux données ECG (166) ;
la sélection (316) pour un tel bloc de la classification à laquelle a été attribuée la valeur la plus élevée ; et
la réalisation d'une analyse des données ECG (166) après que les blocs auxquels a été attribuée la classification de bruit ont été éliminés et l'identification d'au moins un événement cardiaque sur la base de l'analyse.

4. Un système (140) conformément à la revendication 3, où l'unité centrale de traitement applique un seuil de temps à une période de bruit identifié dans chacun parmi un ou plusieurs blocs et attribue une classification de bruit à un tel bloc lorsque la classification de bruit a une valeur plus élevée que la classification valide et que la période du bruit identifié excède le seuil de temps.

5. Un système (140) conformément à la revendication 1, où l'unité centrale de traitement élimine de chaque bloc de données ECG (166) une quantité prédéterminée de données depuis un commencement du bloc de sa prise en considération pendant l'analyse de détection de bruit.

6. Un système (140) conformément à la revendication 1, où le dispositif de surveillance d'électrocardiographie (212) comprend :
un coussinet d'électrode à port prolongé (215) comprenant :

une bandelette allongée (221) ;
une paire d'électrodes électrocardiographiques exposées de façon conductrice sur une surface de contact de chaque extrémité de la bandelette allongée (221),
où une des électrodes électrocardiographiques est collée sur une zone recouvrant le processus xiphoïde sur la poitrine d'un patient et une autre des électrodes électrocardiographiques est collée sur la zone près du manubrium sur la poitrine du patient, orientée de façon centrale (sur la ligne médiane) le long du sternum vers le haut par rapport à cette électrode électrocardiographique ;
un réceptacle non conducteur (225) collé sur une surface tournée vers l'extérieur de la bandelette allongée (221) et comprenant une pluralité de plots électriques (234) ;
une batterie interfacée de façon électrique à une paire des plots électriques (234) sur le réceptacle non conducteur (225) ; et
un circuit (232, 233) fixé sur chaque extrémité de la bandelette allongée (221).

7. Un système (140) conformément à la revendication 6, où le dispositif de surveillance d'électrocardiographie (212) comprend en outre :
un enregistreur de dispositif de surveillance (214) ayant un boîtier scellé (250) conçu pour être assujetti de façon amovible dans le réceptacle non conducteur (225) du coussinet d'électrode, où le boîtier scellé (250) comprend un jeu de contacts électriques (256) qui dépassent d'une surface de dessous et qui correspondent à des plots électriques supplémentaires (234) sur le réceptacle non conducteur (225) et comprenant :

un microcontrôleur à même de fonctionner pour déceler des signaux électrocardiographiques traversant les électrodes électrocardiographiques par l'intermédiaire de la paire des plots électriques (234) ; et
une mémoire flash interfacée de façon électrique avec le microcontrôleur et à même de fonctionner pour stocker des échantillons des signaux électrocardiographiques.

8. Un procédé (270) destiné à la classification de données ECG pour son utilisation dans la facilitation de diagnostics de troubles du rythme cardiaque à l'aide d'un ordinateur numérique, comprenant :

l'obtention de données ECG (166) par l'intermédiaire d'un dispositif de surveillance d'électrocardiographie (212) comprenant un bouton pour patient (255) ;
l'identification au sein des données ECG (166) d'au moins une pression du bouton pour patient (255) ;
la définition (322) au sein des données ECG (166) d'un créneau de bouton comprenant un segment de données avant et après la pression du bouton pour patient (255) ;
la répartition des données ECG (166) en blocs ;
l'application d'une analyse de détection de bruit aux blocs de données ECG (166) ;
l'attribution (318, 319) d'une classification comprenant un élément parmi du bruit et des données valides à chaque bloc de données ECG sur la base de l'analyse de détection de bruit par l'intermédiaire d'un détecteur de bruit entraîné ;
l'identification (324) d'au moins un bloc de données ECG (166) auquel a été attribuée la classification de bruit qui chevauche le créneau de bouton ;
la modification d'une désignation du bloc de données ECG de la classification de bruit en la classification valide afin de s'assurer que les données ECG dans ce bloc soient revues par la suite ;
la troncation (325) de l'au moins un bloc de données ECG (166) afin qu'il s'aligne sur un élément parmi le début et la fin du créneau de bouton ;
le maintien du bloc de données ECG tronqué comme données valides ; et
l'élimination (326) des données ECG (166) des blocs et des blocs tronqués auxquels la classification de bruit a été attribuée.

**9.** Un procédé (270) conformément à la revendication 8, comprenant en outre :

la réalisation d'une analyse des données ECG (166) après que les blocs auxquels a été attribuée la classification de bruit ont été éliminés ; et
l'identification d'au moins un événement cardiaque sur la base de l'analyse.

**10.** Un procédé (270) conformément à la revendication 8, comprenant en outre :
la réalisation de l'analyse de détection de bruit, comprenant :

l'identification (312) de caractéristiques ;
l'identification (313) de motifs répétitifs des caractéristiques ;
la génération (314) d'une matrice avec les caractéristiques et motifs identifiés ;
la génération d'au moins une matrice avec des poids pour les caractéristiques et motifs identifiés ; et
l'attribution (318, 319) d'une valeur pour chacune parmi la classification de bruit et la classification valide à chaque bloc sur la base de la matrice de poids.

**11.** Un procédé (270) conformément à la revendication 10, comprenant en outre au moins une action parmi :

la détermination (312) des caractéristiques par application d'un ou de plusieurs filtres glissants aux données ECG (166) ; et
la sélection (316) pour un tel bloc de la classification à laquelle a été attribuée la valeur la plus élevée.

**12.** Un procédé (270) conformément à la revendication 11, comprenant en outre :

l'application d'un seuil de temps à une période de bruit identifié dans chacun parmi un ou plusieurs blocs ; et
l'attribution d'une classification de bruit à un tel bloc lorsque la classification de bruit a une valeur plus élevée que la classification valide et que la période de bruit identifié excède le seuil de temps.

**13.** Un procédé (270) conformément à la revendication 8, comprenant en outre :
de chaque bloc de données ECG (166), l'élimination d'une quantité prédéterminée de données depuis un commencement du bloc de sa prise en considération pendant l'analyse de détection de bruit.

**14.** Un procédé (270) conformément à la revendication 8, où le dispositif de surveillance d'électrocardiographie (212) comprend :
un coussinet d'électrode à port prolongé (215) comprenant :

une bandelette allongée (221) ;
une paire d'électrodes électrocardiographiques exposées de façon conductrice sur une surface de contact de chaque extrémité de la bandelette allongée (221), où une des électrodes électrocardiographiques est collée sur une zone recouvrant le processus xiphoïde sur la poitrine d'un patient et une autre des électrodes électrocardiographiques est collée sur la zone près du manubrium sur la poitrine du patient, orientée de façon centrale (sur la ligne médiane) le long du sternum vers le haut par rapport à cette électrode électrocardiographique ;
un réceptacle non conducteur (225) collé à une surface tournée vers l'extérieur de la bandelette allongée (221) et comprenant une pluralité de plots électriques (234) ;
une batterie interfacée de façon électrique à une paire des plots électriques (234) sur le réceptacle non conducteur (225) ; et

un circuit (232, 233) fixé sur chaque extrémité de la bandelette allongée (221).

**15.** Un procédé (270) conformément à la revendication 14, où le dispositif de surveillance d'électrocardiographie (212) comprend en outre :
un enregistreur de dispositif de surveillance (214) ayant un boîtier scellé (250) conçu pour être assujetti de façon amovible dans le réceptacle non conducteur (225) du coussinet d'électrode (215), où le boîtier scellé (250) comprend un jeu de contacts électriques (256) qui dépassent d'une surface de dessous et qui correspondent à des plots électriques supplémentaires (234) sur le réceptacle non conducteur (225) et comprenant :

un microcontrôleur à même de fonctionner pour déceler des signaux électrocardiographiques traversant les électrodes électrocardiographiques par l'intermédiaire des plots électriques (234) ; et
une mémoire flash interfacée de façon électrique avec le microcontrôleur et à même de fonctionner pour stocker des échantillons des signaux électrocardiographiques.

# Fig. 1.

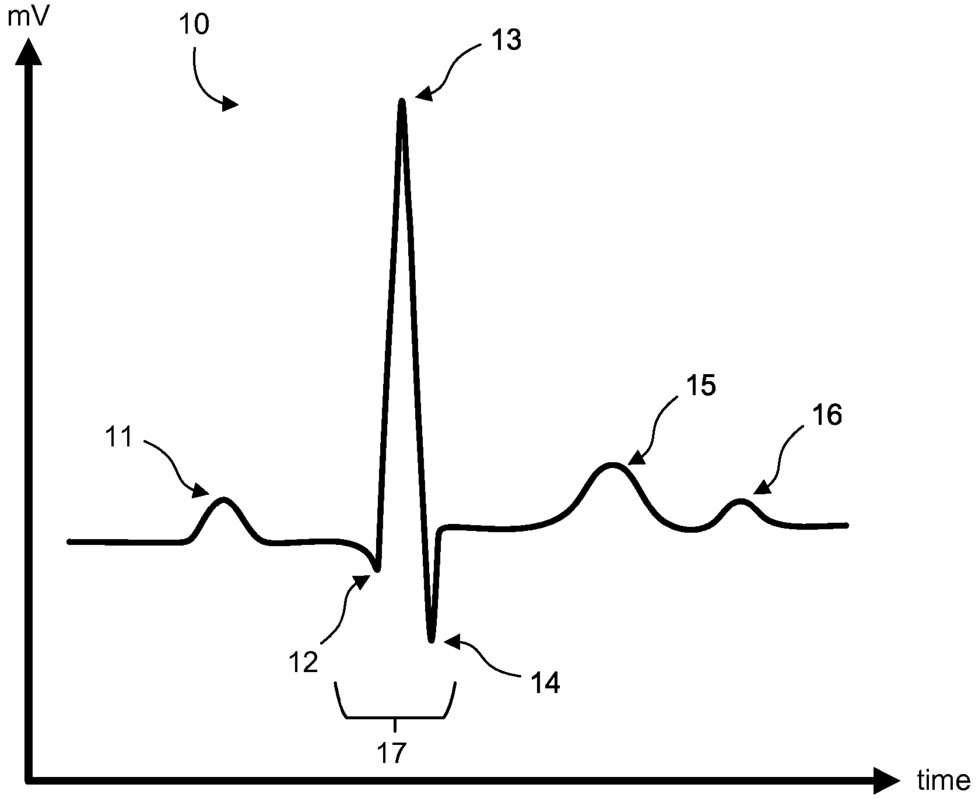


# Fig. 2. (Prior Art)

18

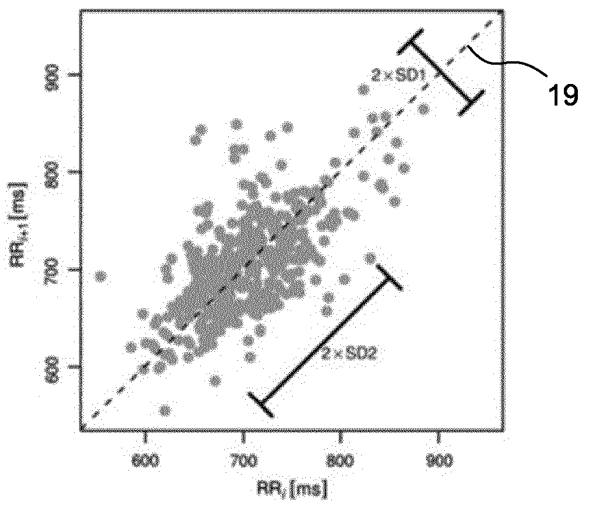

Fig. 3.

20

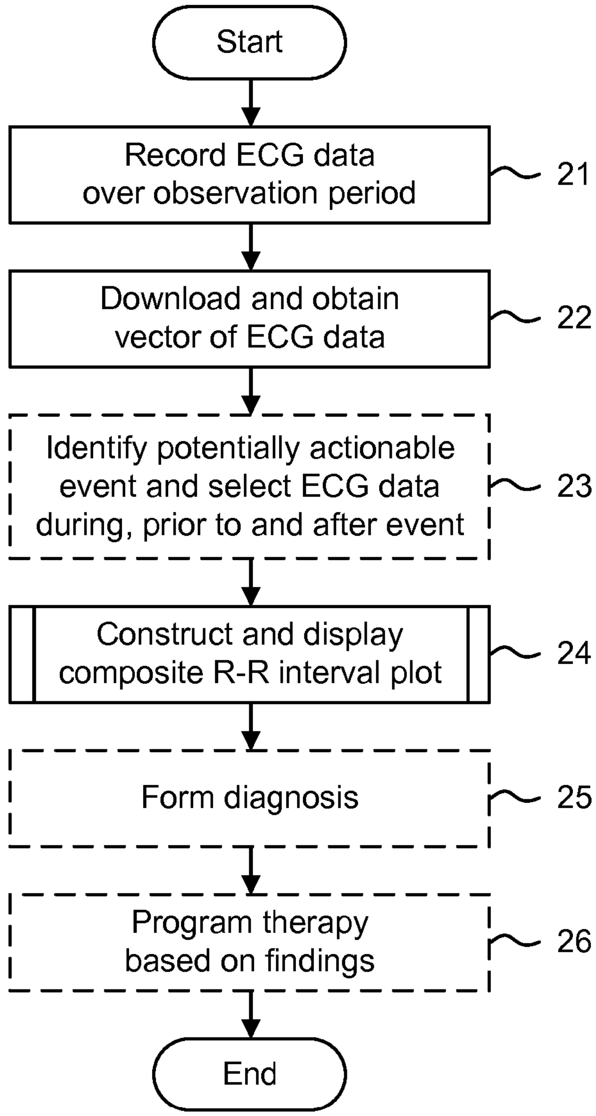
Start
Record ECG data over observation period
21
Download and obtain vector of ECG data
22
Identify potentially actionable event and select ECG data during, prior to and after event
23
Construct and display composite R-R interval plot
24
Form diagnosis
25
Program therapy based on findings
26
End

Fig. 4.

30

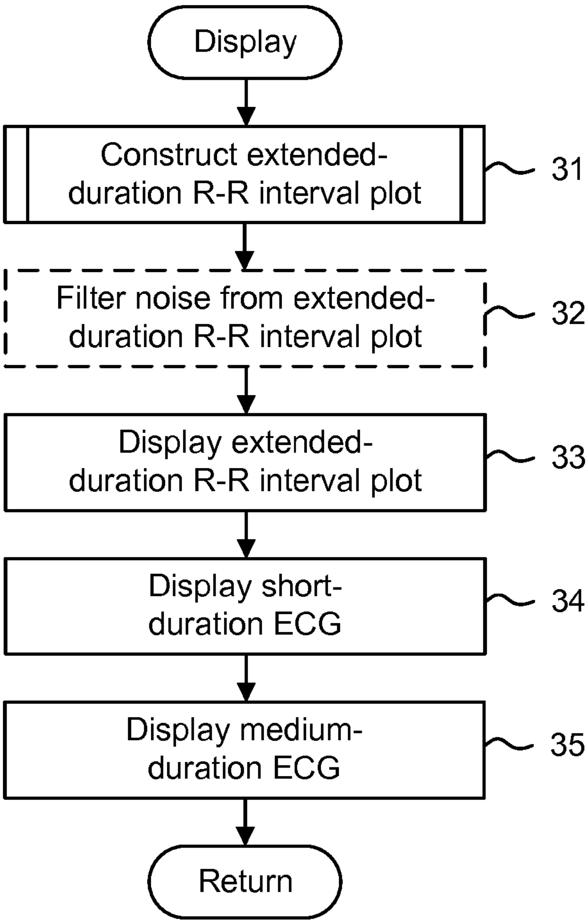
Display
Construct extended-duration R-R interval plot
31
Filter noise from extended-duration R-R interval plot
32
Display extended-duration R-R interval plot
33
Display short-duration ECG
34
Display medium-duration ECG
35
Return

# Fig. 5.

40

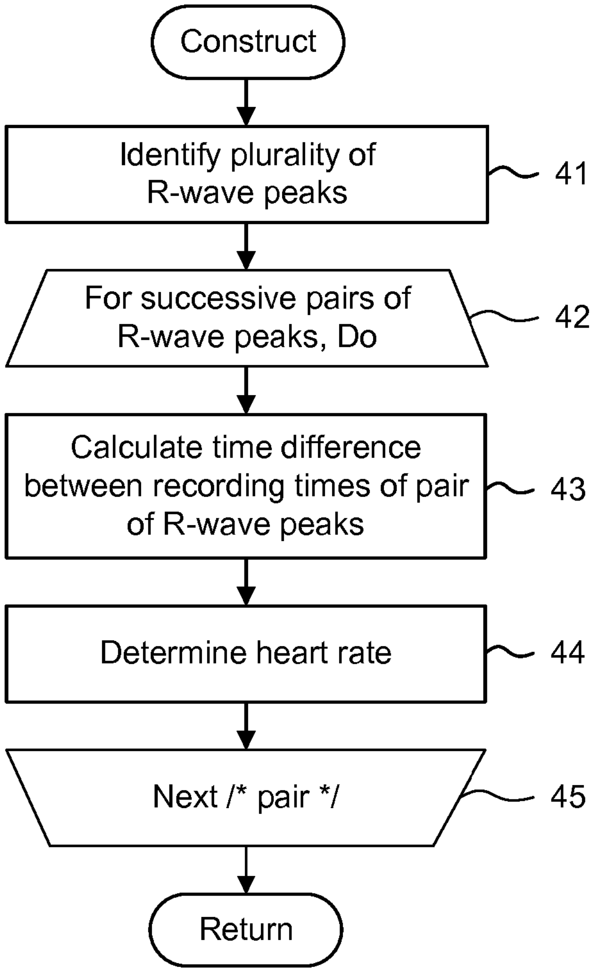
Construct
Identify plurality of R-wave peaks
41
For successive pairs of R-wave peaks, Do
42
Calculate time difference between recording times of pair of R-wave peaks
43
Determine heart rate
44
Next /* pair */
45
Return

# Fig. 6.

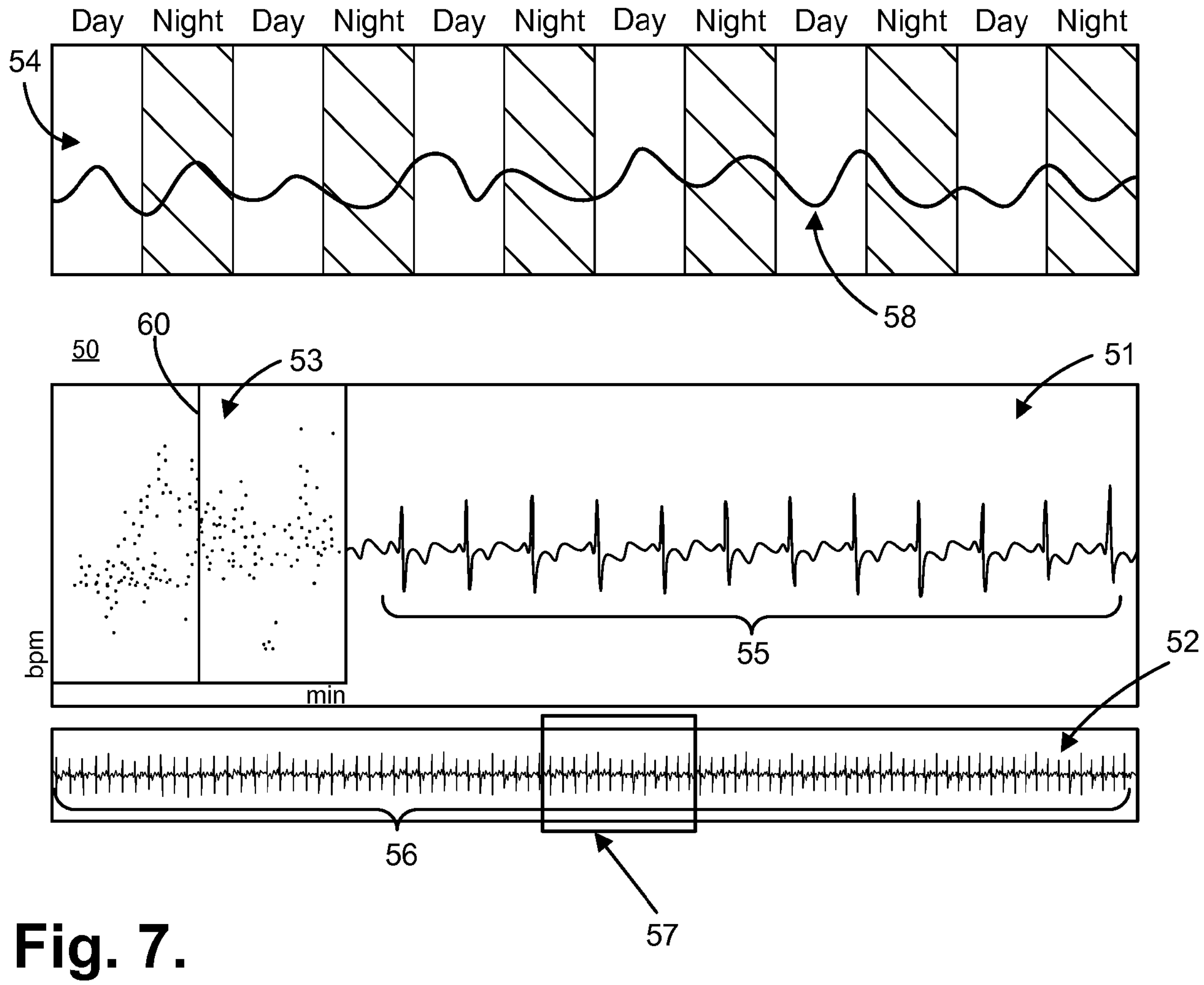
Day
Night
Day
Night
Day
Night
Day
Night
Day
Night
Day
Night
54
58
50
60
53
51
55
bpm
min
52
56
57

# Fig. 7.

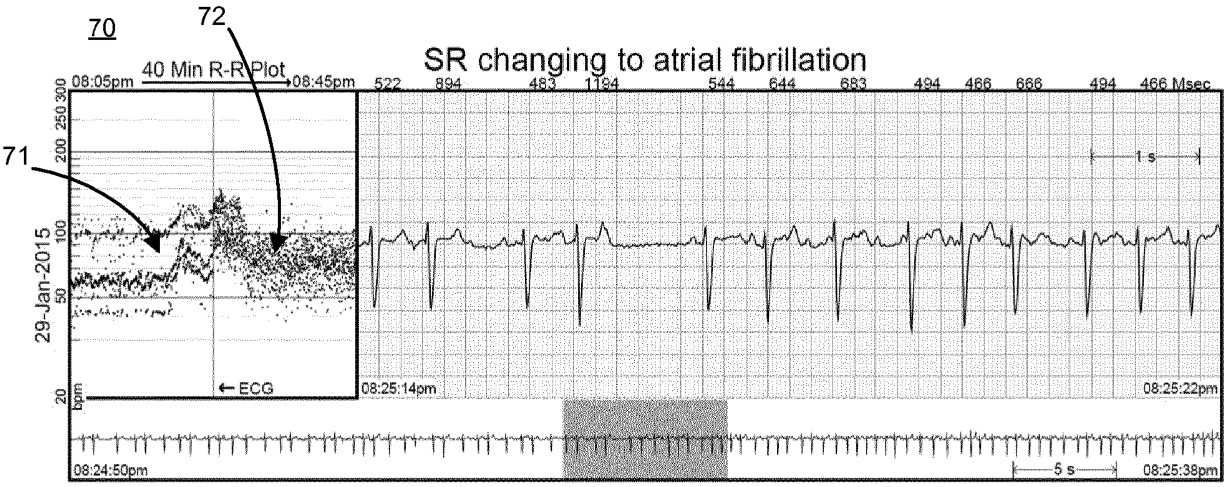
70
72
40 Min R-R Plot
08:05pm
08:45pm
SR changing to atrial fibrillation
522
894
483
1194
544
644
683
494
466
666
494
466 Msec
71
300
250
200
100
50
20
bpm
29-Jan-2015
← ECG
1 s
08:25:14pm
08:25:22pm
08:24:50pm
5 s
08:25:38pm

# Fig. 8.

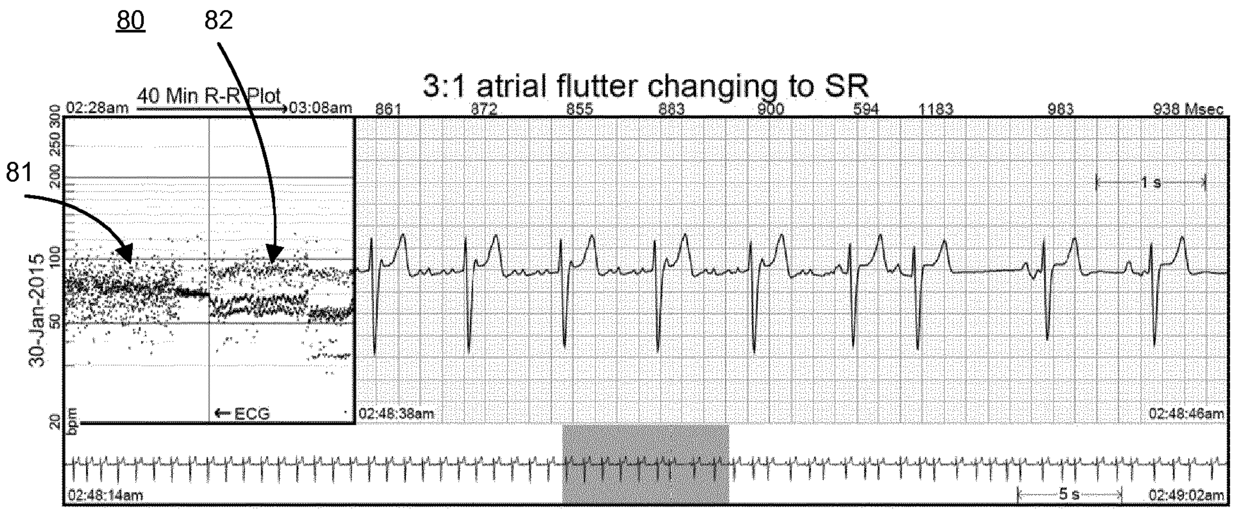
80
82
81
3:1 atrial flutter changing to SR
40 Min R-R Plot
02:28am
03:08am
861 872 855 883 900 594 1183 983 938 Msec
30-Jan-2015
300 250 200 100 50 20
bpm
← ECG
1 s
02:48:38am
02:48:46am
02:48:14am
5 s
02:49:02am

# Fig. 9.

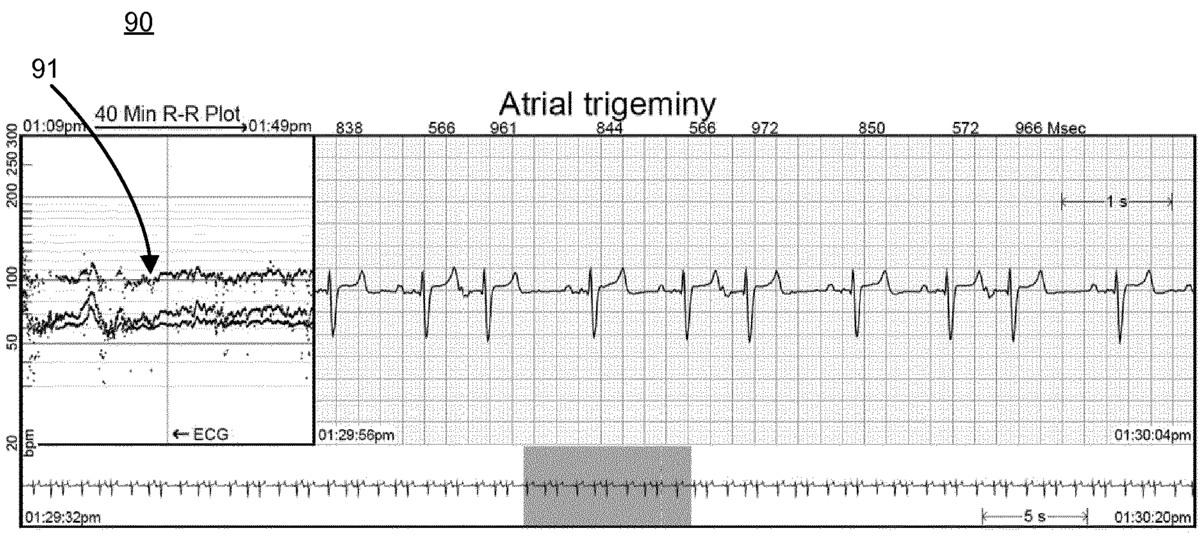
90
91
Atrial trigeminy
40 Min R-R Plot
01:09pm
01:49pm
838 566 961 844 566 972 850 572 966 Msec
300 250 200 100 50 20
bpm
← ECG
1 s
01:29:56pm
01:30:04pm
01:29:32pm
5 s
01:30:20pm

# Fig. 10.

100

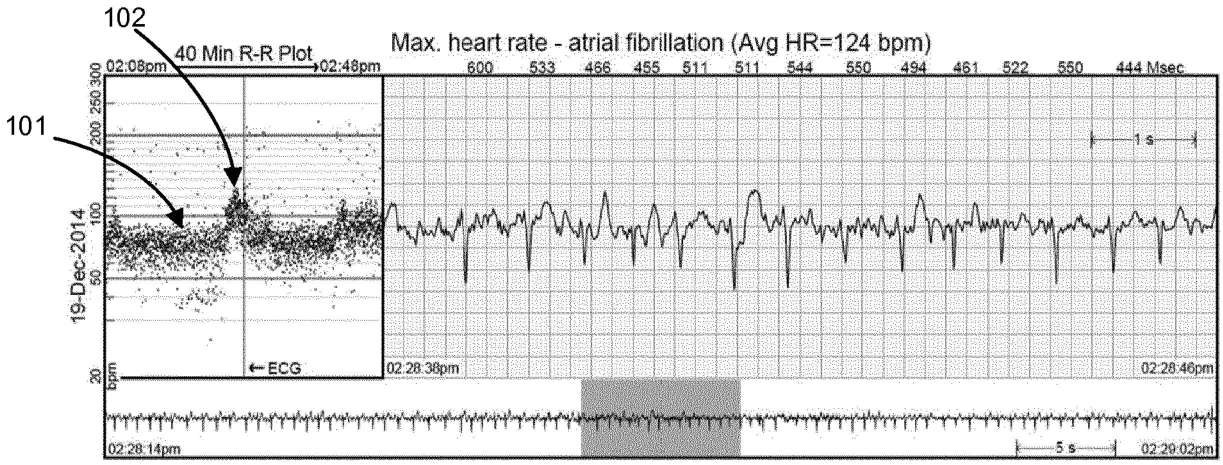


# Fig. 11.

110

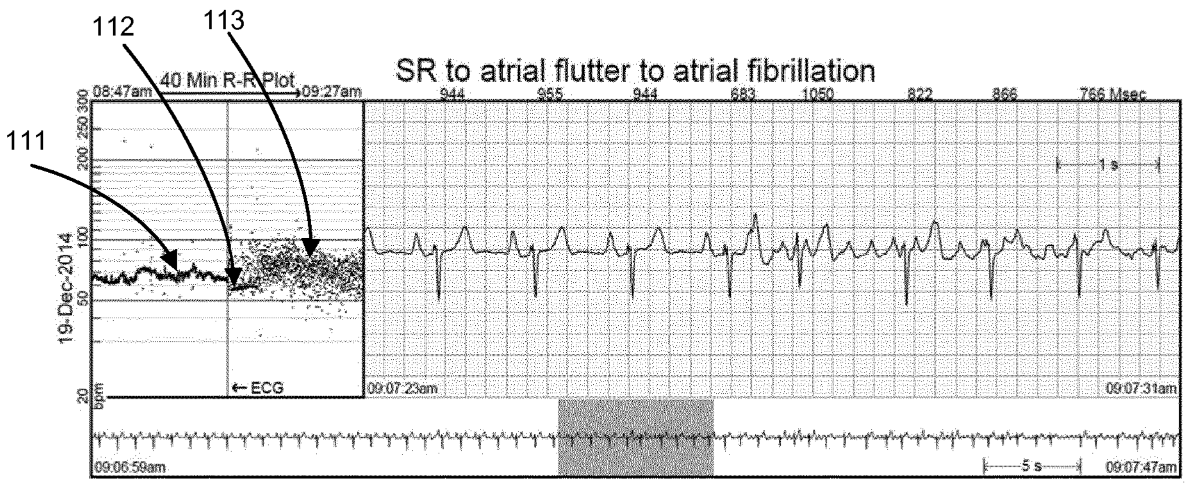

# Fig. 12.

120

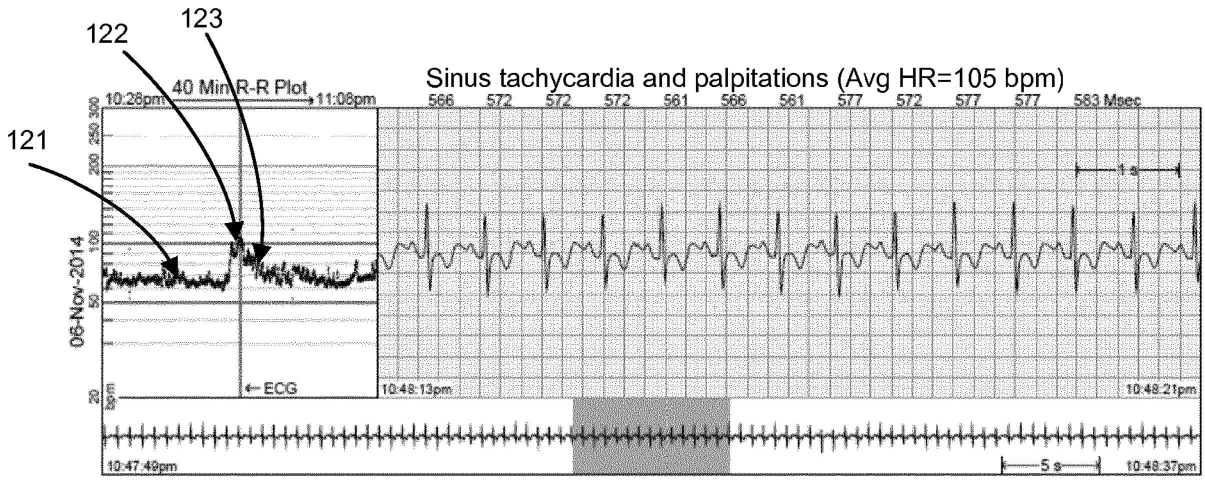


# Fig. 13.

130

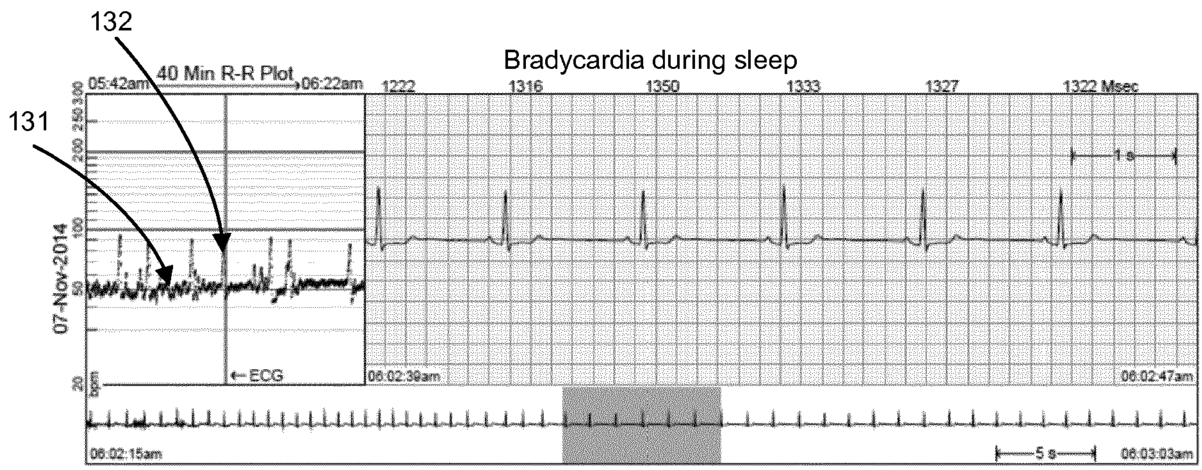

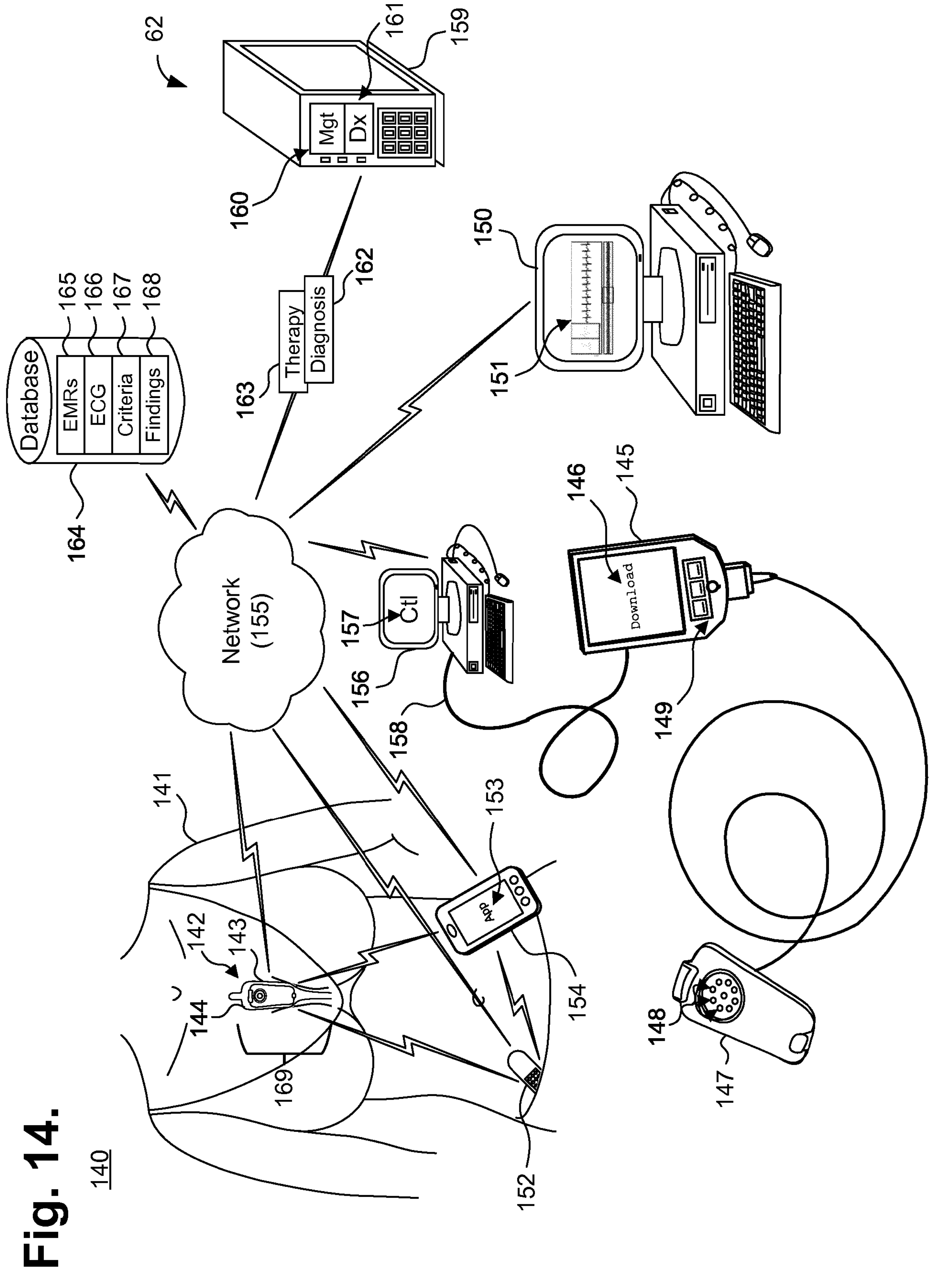
62
159
161
Mgt
Dx
160
Database
EMRs
ECG
Criteria
Findings
165
166
167
168
164
Therapy
Diagnosis
162
163
150
151
Network
(155)
157
Ctl
156
158
146
145
Download
149
141
142
143
144
169
153
App
154
152
148
147

Fig. 14.
140

Fig. 15.

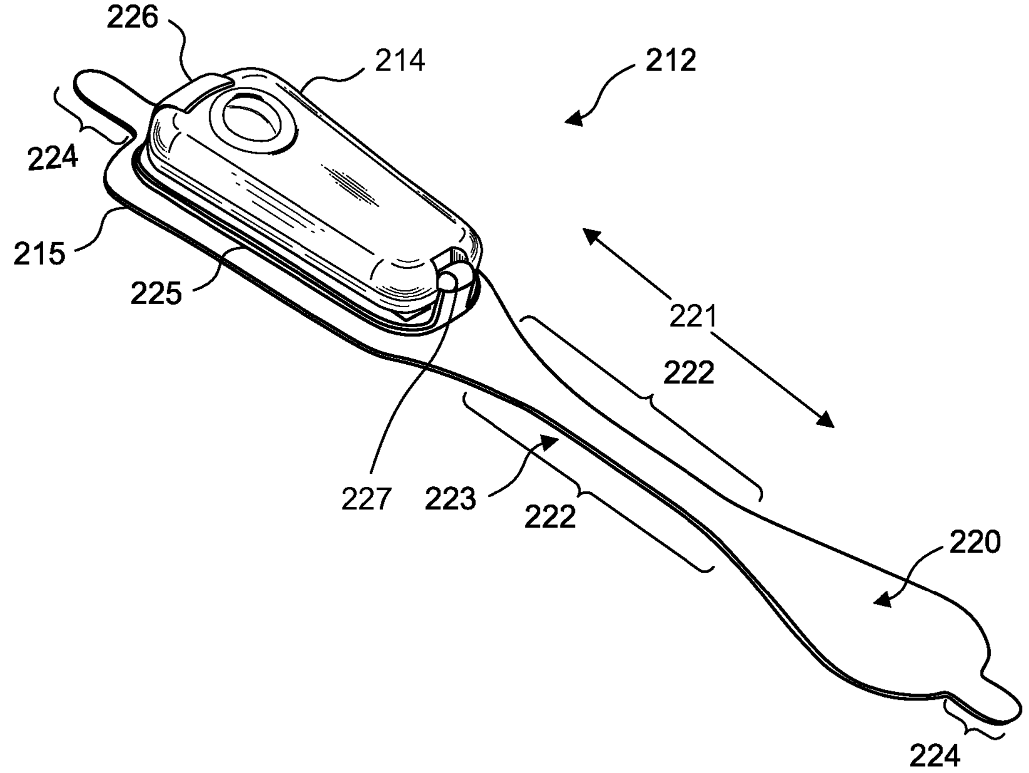
226
214
212
224
215
225
221
222
227
223
222
220
224

Fig. 16.

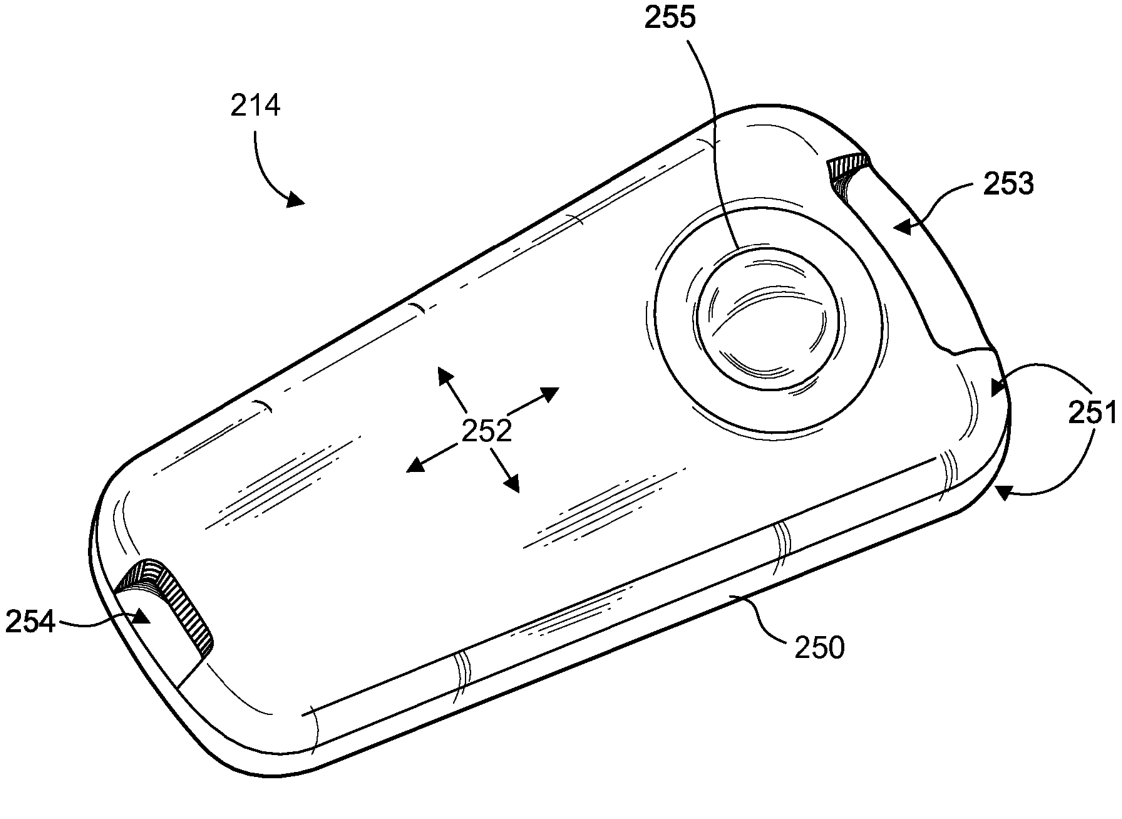
255
214
253
252
251
254
250

Fig. 17.

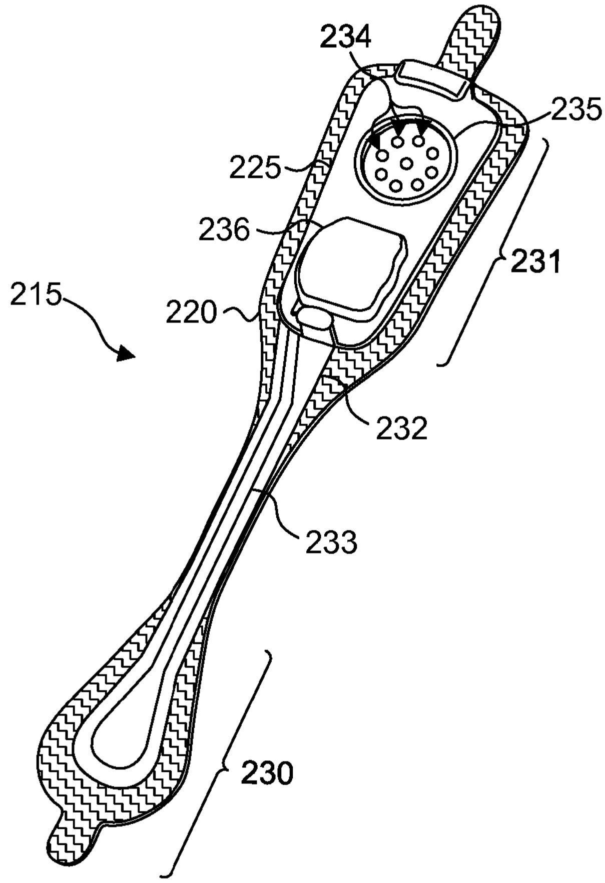


Fig. 18.

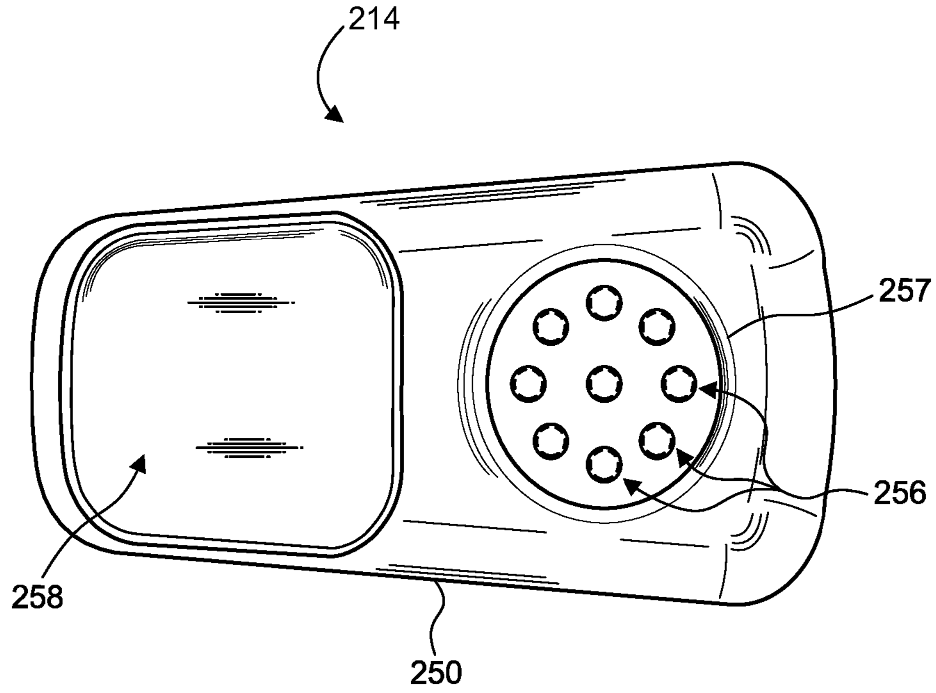

Fig. 19.

270

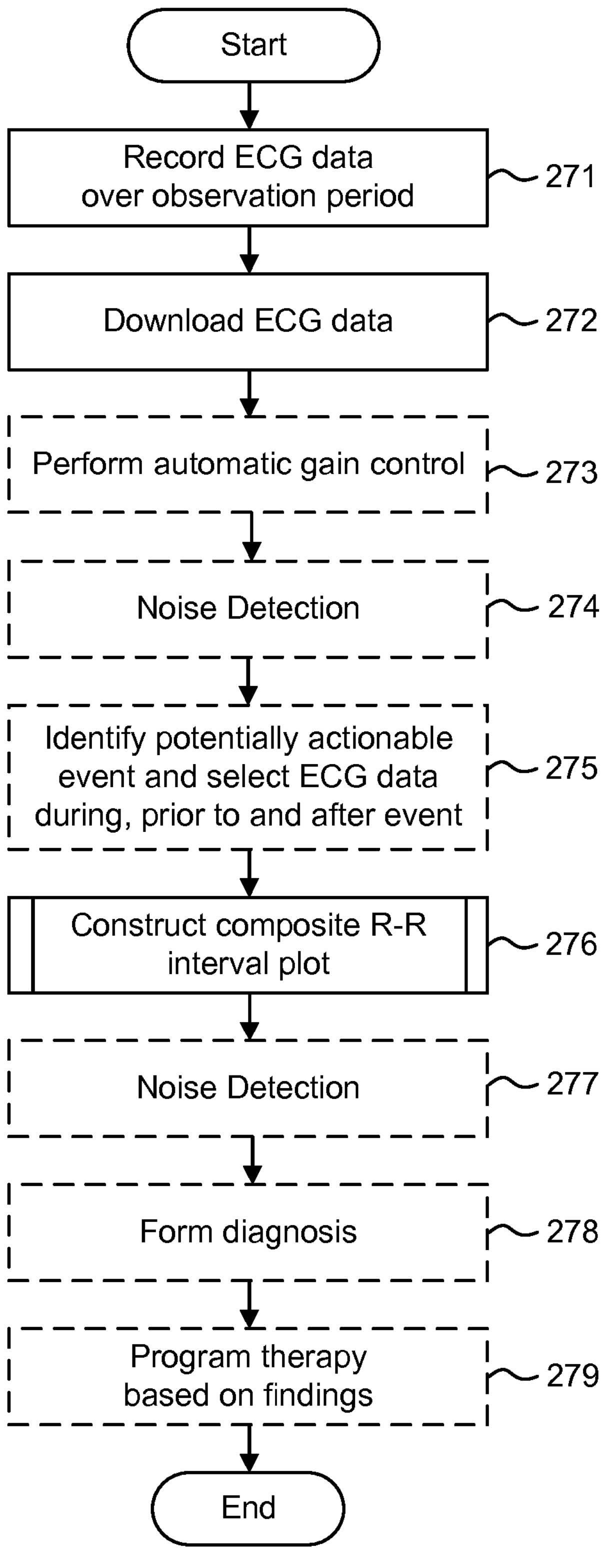
Start
Record ECG data over observation period
271
Download ECG data
272
Perform automatic gain control
273
Noise Detection
274
Identify potentially actionable event and select ECG data during, prior to and after event
275
Construct composite R-R interval plot
276
Noise Detection
277
Form diagnosis
278
Program therapy based on findings
279
End

**Fig. 20.**

290

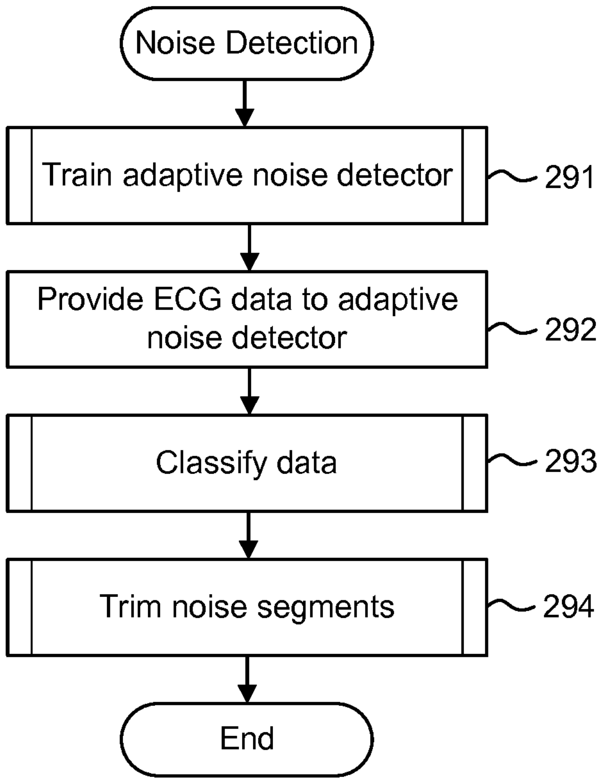
Noise Detection
Train adaptive noise detector
291
Provide ECG data to adaptive noise detector
292
Classify data
293
Trim noise segments
294
End

# Fig. 21.

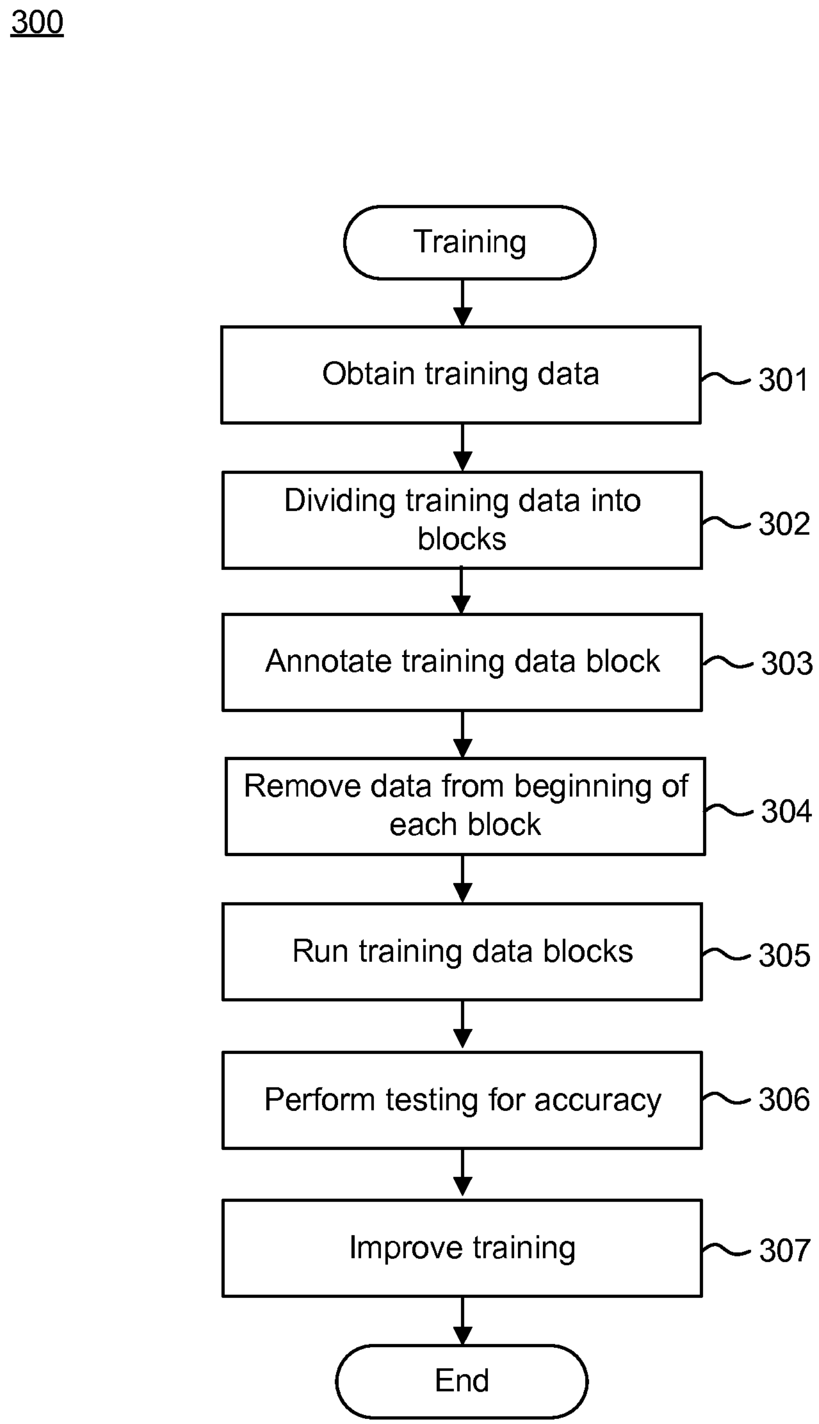
300
Training
Obtain training data
301
Dividing training data into blocks
302
Annotate training data block
303
Remove data from beginning of each block
304
Run training data blocks
305
Perform testing for accuracy
306
Improve training
307
End

# Fig. 22.

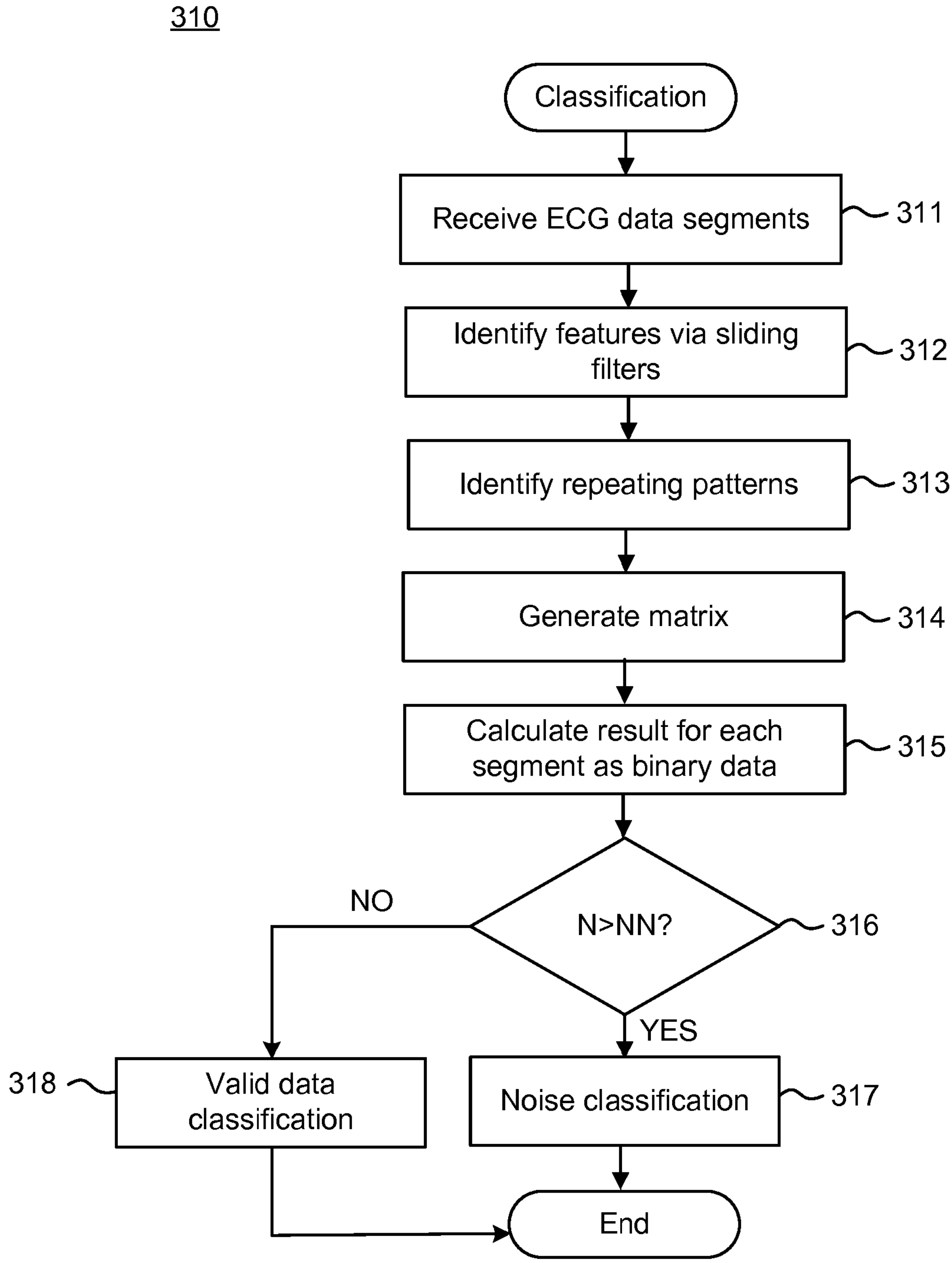
310
Classification
Receive ECG data segments
311
Identify features via sliding filters
312
Identify repeating patterns
313
Generate matrix
314
Calculate result for each segment as binary data
315
N>NN?
316
NO
YES
318
Valid data classification
Noise classification
317
End

Fig. 23.

320

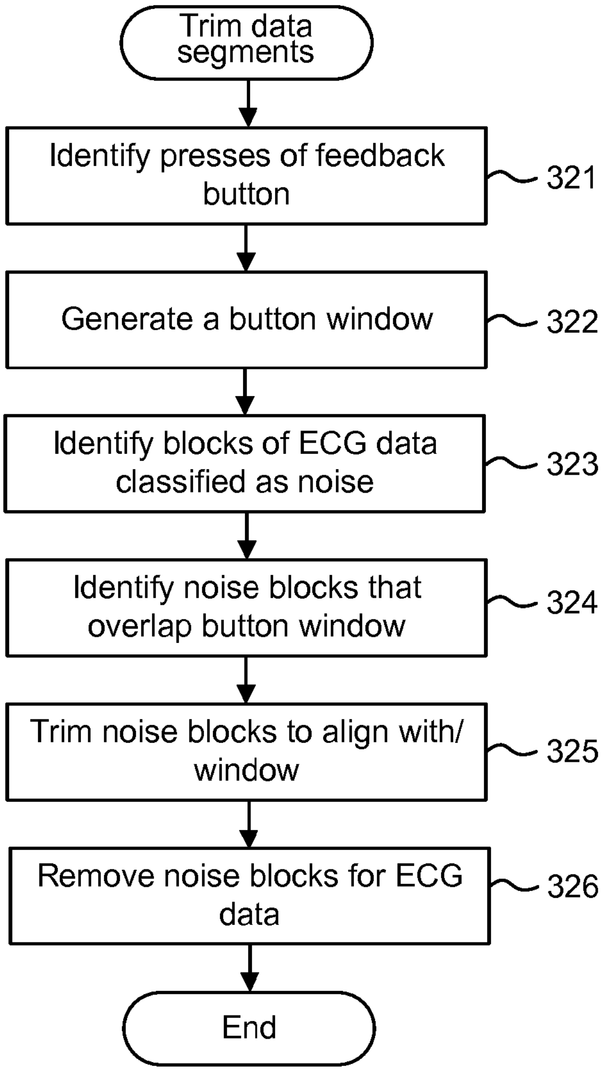
Trim data segments
Identify presses of feedback button
321
Generate a button window
322
Identify blocks of ECG data classified as noise
323
Identify noise blocks that overlap button window
324
Trim noise blocks to align with/ window
325
Remove noise blocks for ECG data
326
End

# Fig. 24.

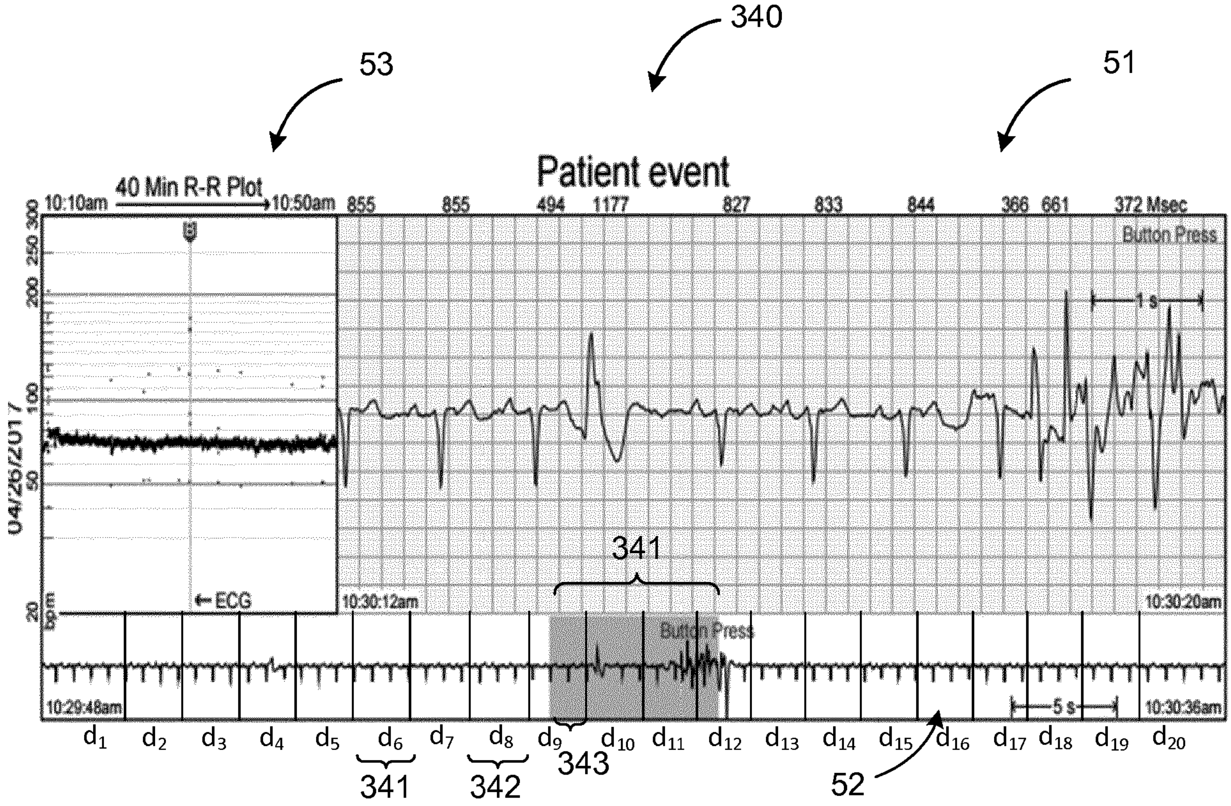
340
53
51
Patient event
40 Min R-R Plot
10:10am
10:50am
855
855
494
1177
827
833
844
366
661
372 Msec
Button Press
1 s
04/26/2017
bpm
ECG
10:30:12am
10:30:20am
341
Button Press
10:29:48am
5 s
10:30:36am
d1 d2 d3 d4 d5 d6 d7 d8 d9 d10 d11 d12 d13 d14 d15 d16 d17 d18 d19 d20
341
342
343
52

# Fig. 25.

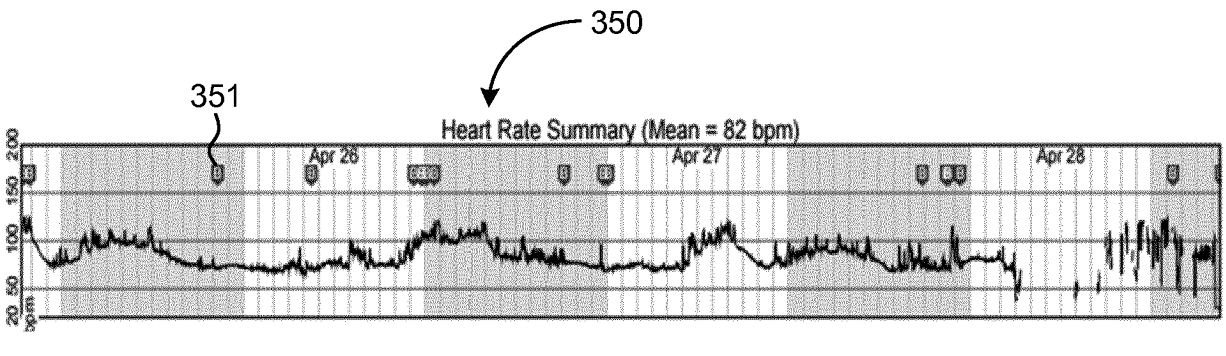
350
351
Heart Rate Summary (Mean = 82 bpm)
Apr 26
Apr 27
Apr 28
bpm

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2017196472 A1
- US 9345414 B
- US 744659 D
- US 9545204 B
- US 717955 D